# EUROPÄISCHE PATENTSCHRIFT

(11) **EP 1 077 245 B1**
(45) Veröffentlichungstag und Bekanntmachung des Hinweises auf die Patenterteilung: **07.01.2004**
(21) Anmeldenummer: 00116293.2
(22) Anmeldetag: 10.08.2000
(51) Int. Cl.: C09K 15/20, C09K 15/24, C07C 7/20, C07B 63/04

(54) **Inhibitorkomposition zur Stabilisierung von radikalisch polymerisierbaren Substanzen**
Inhibitor composition for stabilising radically polymerisable substances
Composition inhibante destinée à la stabilisation de substances radicalement polymèrisables

(30) Priorität: 17.08.1999 DE 19938841
(43) Veröffentlichungstag der Anmeldung: 21.02.2001
(73) Patentinhaber: BASF AKTIENGESELLSCHAFT, 67056 Ludwigshafen (DE)
(72) Erfinder: Sutoris, Heinz Friedrich, Dr., 67551 Worms (DE); Wagenblast, Gerhard, Dr., 67157 Wachenheim (DE); Schliephake, Volker, Dr., 67105 Schifferstadt (DE); Schröder, Jürgen, Dr., 67071 Ludwigshafen (DE); Keller, Harald, Dr., 67069 Ludwigshafen (DE); Jaworek, Thomas, Dr., 67169 Kallstadt (DE)
(74) Vertreter: Isenbruck, Günter, Dr.

(56) Entgegenhaltungen:
- EP-A- 0 620 206
- WO-A-00/36052
- DE-A- 19 651 307
- DE-A- 19 707 151
- US-A- 3 818 079
- US-A- 5 322 960

## Beschreibung

Die Erfindung betrifft eine Inhibitorkomposition, sowie deren Verwendung zur Stabilisierung von radikalisch polymerisierbaren Substanzen und ein die Inhibitorkomposition enthaltendes Gemisch.

Viele Verbindungen, die ein oder mehrere vinylisch ungesättigte Gruppen aufweisen, haben eine ausgeprägte Neigung zur radikalischen Polymerisation. Solche Verbindungen werden als Monomere für die radikalische Polymerisation auch gezielt eingesetzt. Gleichzeitig ist die ausgeprägte Neigung zur radikalischen Polymerisation aber insofern von Nachteil, als es sowohl bei der Lagerung als auch bei der chemischen und/oder physikalischen Bearbeitung (z.B. Destillation oder Rektifikation), insbesondere unter der Einwirkung von Wärme und/oder Licht, zur unerwünschten radikalischen Polymerisation der vinylisch unge-sättigten Verbindungen kommen kann. Eine unerwünschte radikalische Polymerisation kann, insbesondere falls Polymer abgeschieden wird, sich in unterschiedlicher Weise negativ auswirken. Beispielsweise kann sich bei der Destillation von vinylisch ungesättigten Verbindungen durch radikalische Polymerisation gebildetes Polymer auf der Oberfläche des eingesetzten Verdampfers niederschlagen - dort ist die Neigung zur radikalischen Polymerisation infolge der hohen Temperaturen besonders stark ausgeprägt. Polymerisation im Bereich der Oberfläche eines Verdampfers bedeutet in der Regel, daß sich eine Polymerschicht an der Oberfläche ausbildet. Aufgrund der isolierenden Wirkung der Polymerschicht wird der Wärmeübergang in unerwünschter Weise vermindert. Durch radikalische Polymerisation unerwünscht gebildetes Polymer kann aber auch die Einbauten von Rektifikationskolonnen verstopfen, was unerwünschte Druckverluste verursacht. Die Abscheidung von Polymer kann letztendlich das Unterbrechen des Rektifikationsprozesses erforderlich machen, da zur Fortführung der Rektifikation das abgeschiedene Polymer entfernt werden muß.

Es ist daher allgemeine Praxis, vinylisch ungesättigten Verbindungen, die radikalisch polymerisierbar sind und Gemischen, die solche Verbindungen enthalten, Verbindungen zuzusetzen, die als Inhibitoren bzw. Retarder der radikalischen Polymerisation fungieren. Während Inhibitoren die radikalische Polymerisation - bis zu deren vollständigen Umsetzung mit freien Radikalen - unterbinden, verlangsamen Retarder die radikalische Polymerisation. Inhibitoren und Retarder werden im allgemeinen unter dem Oberbegriff Stabilisatoren zusammengefaßt. Im folgenden sollen jedoch sowohl Inhibitoren als auch Retarder als Inhibitoren verstanden werden. Sowohl bei der Lagerung als auch bei der chemisch und/oder physikalischen Behandlung (zum Beispiel bei der Destillation) von vinylisch ungesättigten Verbindungen, die radikalisch polymerisierbar sind, ist der Einsatz von Inhibitoren bzw. Retardem von Bedeutung.

Die US-A 4,187,382 betrifft ein Verfahren zur Veresterung von organischen Diolen mit Acrylsäure. Es wird empfohlen, das Diol mit Triphenylphosphit vorzubehandeln, um so die Neigung des Reaktionsgemisches zur radikalischen Polymerisation zu verringern. Als weitere inhibierende Komponente wird ein phenolischer Polymerisationsinhibitor vorgeschlagen.

Die DE-A 29 13 218 offenbart ein Verfahren zur Herstellung von Acrylsäureestern bzw. Methacrylsäureestem, bei dem als Polymerisationsinhibitor organische Phosphite zusammen mit phenolischen Polymerisationsinhibitoren eingesetzt werden.

Vorstehend genannte Inhibitorkompositionen enthalten Phosphorverbindungen, die Phosphor in der Oxidationszahl +3 aufweisen und außerdem phenolische Verbindungen. Eine Zielsetzung ist, die Wirkung solcher Systeme bei der Stabilisierung von vinylisch ungesättigten Verbindungen weiter zu verbessern.

Die Aufgabe der vorliegenden Erfindung besteht darin, eine stark wirkende Inhibitorkomposition hervorzubringen, die Phosphor in der Oxidationszahl +3 aufweisende chemische Verbindungen und/oder phenolische Verbindungen enthält. Diese Inhibitorkomposition soll effektiv sein, wobei sich möglichst die inhibierende Wirkung der in ihr enthaltenen Komponenten in synergistischer Weise verstärken soll.

Gelöst wird diese Aufgabe durch die Bereitstellung einer Inhibitorkomposition enthaltend als Komponenten a) wenigstens ein Nitroxyl-Radikal(derivat), b) wenigstens ein Phenol(derivat) und c) wenigstens eine weitere chemische Verbindung, welche mindestens ein Phosphoratom enthält, das die Oxidationszahl +3 aufweist.

Unter der Oxidationszahl eines Atoms innerhalb einer kovalenten Verbindung soll eine Zahl mit positivem oder negativem Vorzeichen verstanden werden, die die Ladung angibt, welches das Atom haben würde, wenn man die bindenden Elektronenpaare der kovalenten Bindungen, an denen das Atom teilnimmt, dem jeweils elektronegativeren Bindungspartner zuteilt. Bei Elektronenpaaren aus kovalenten Bindungen zwischen zwei gleichen Atomen enthält jedes Atom ein Elektron. Die Elektronegativität ist dabei als Maß anzusehen, wie stark ein Atom in einem Molekül bindende Elektronenpaare, die an dem Atom gebunden sind, anzieht. Die vorliegend relevanten Elektronegativitäten sind diejenigen gemäß H.R. Christen, Grundlagen der allgemeinen und anorganischen Chemie, Verlag Sauerländer, Aarau, Diesterweg-Salle, Frankfurt am Main (1973). Für die wichtigsten Elemente des Periodensystems weisen diese Elektronegativitäten die nachfolgenden Werte auf:
Be (1,5) ; B (2,0) ; H (2,1) ; C (2,5) ; Si (1,8) ; Ge (1,7) ; N (3,0) ; P (2,1) ; As (2,0) ; Sb (1,8) ; O (3,5) ; S (2,5) ; Se (2,4) ; Te (2,1); F (4,0) ; Cl (3,0) ; Br (2,8) ; J (2,4).

Als Verbindungen (Komponente c)), welche mindestens ein die Oxidationszahl +3 aufweisendes Phosphoratom enthalten, können insbesondere Orthophos-phorige Säure oder ein Ester der Orthophosphorigen Säure eingesetzt werden. Ester der Orthophosphorigen Säure werden auch als Phosphite bezeichnet. Die Orthophosphorige Säure kann auch als Salz (meist als Alkalimetall- oder Ammoniumsalz) vorliegen. Bevorzugte Bindungspartner des Phosphors sind die Elemente C, S, O, N und/oder H.

Ferner kommen - insbesondere die als Stabilisatoren bekannten - Phosphonite (Ester der Phosphonigsäure) in Betracht.

Zu besonders geeigneten Phosphiten (also der Ester der Orthophosphorigen Säure) und Phosphoniten (Ester der Phosphonigsäure) zählen beispielsweise Triphenylphosphit, Diphenylalkylphosphit, Phenyldialkylphosphit, Tris(nonylphenyl)phosphit, Trilaurylphosphit, Trioctadecylphosphit, Distearylphentaerythritoldiphosphit, Tris(2,4-ditert.-butylphenyl)phosphit, Diisodecypentaerythritoldiphosphit, Bis(2,4-di-tert.butylphenyl)pentaerythritol-diphosphit, Bis(2,6-di-tert-butyl-4-methylphenyl)-pentaerythritoldiphosphit, Diisodecyloxypentaerythritoldiphosphit, Bis(2,4-di-tert.-butyl-6-methylphenyl)pentaerythritoldiphosphit, Bis(2,4,6-tris-(tertbutylphenyl))pentaerythritoldiphosphit, Tristearylsorbitoltriphosphit, Tetrakis(2,4-di-tert.butylphenyl)-4,4'-biphenylendiphosphit, Tetrakis(2,4-di-tert.-butylphenyl)-4,4'biphenylendiphosphonit, 6-Isooctyloxy-2,4,8,10-tetra-tert.-butyl-12H-dibenz-[d,g]-1,3,2-dioxaphosphocin, 6-Fluoro-2,4,8,10-tetra-tert.-butyl12-methyl-dibenz[d,g]-1,3,2-dioxaphosphocin, Bis(2,4-di-tert.-butyl-6-methylphenyl)methylphosphit und Bis(2,4-ditert.-butyl-6-methylphenyl)ethylphosphit.
Mit Vorteil werden dabei Ester der Orthophosphorigen Säure (Phosphite) der allgemeinen Formel (I) oder Ester der Phosphonigsäure (Phosphonite) der allgemeinen Formel (II) 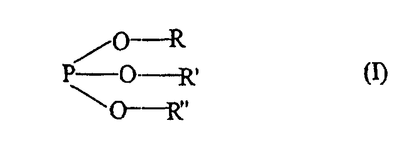 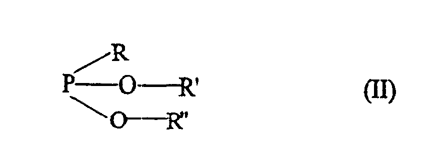 eingesetzt,
wobei R, R', R" gleich oder verschieden sein können und organische Reste insbesondere C₁-C₂₀-Alkyl, Hydroxy-C₂-C₄-alkyl, Halogen-C₂-C₄-alkyl, insbesondere Chloralkyl, -C₆-C₁₀-Aryl, insbesondere Phenyl oder durch C₁-C₈-Alkyl substituiertes Aryl (insbesondere durch C₁-C₄-Alkyl-substituiertes Phenyl) bedeuten. Auch können zwei der drei organischen Reste R, R' und R" gemeinsam mit dem Phosphor und den beiden Sauerstoffatomen einen Heterocyclus (zum Beispiel 5- oder 6-atomig) bilden.

Namentlich genannt seien Trimethyl-, Triethyl-, Tributyl-, Trihexyl-, Trioctyl-, Triphenyl, Tri-p-kresyl-, Trixylyl-, Tritolyl- und Tri-β-chlorethylphosphit. Aber auch Dimethyl-, Diethyl-, Dibutyl-, Dioctyl-, Diphenyl-, Ditolyl- und Dixylylphosphite sind erfindungsgemäß geeignete Inhibitoren. Besonders geeignet sind die unter den Markennamen Irgafos® 168 (Hersteller Ciba AG), Irgafos® P-EPQ (Hersteller Ciba AG) oder Ultranox® 626 (Hersteller GE-Speciality Chemicals GmbH) bekannten Spezies:

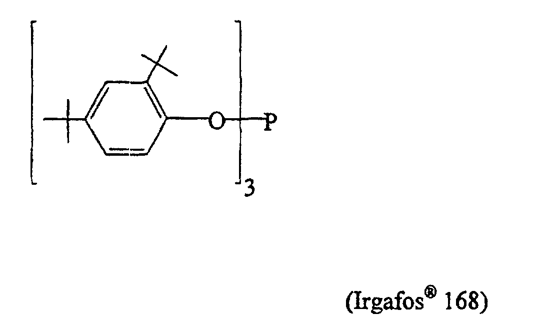

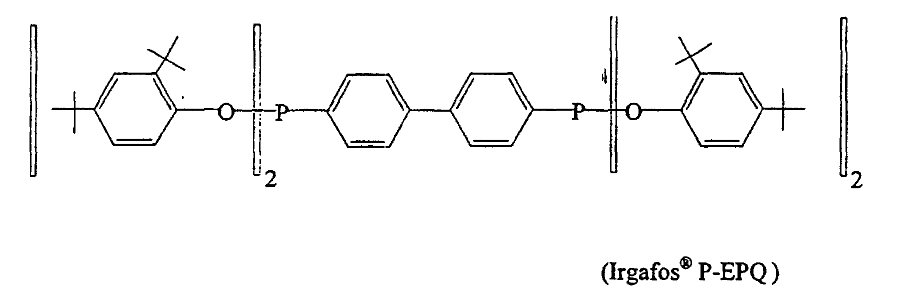

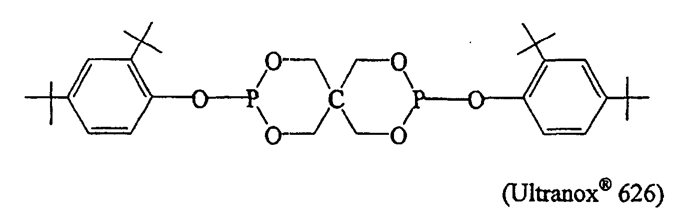

Außerdem eignen sich als chemische Verbindungen, welche mindestens ein Phosphoratom enthalten, das die Oxidationszahl +3 aufweist, die Derivate der Phosphonigsäure R*-P(OH)₂, mit R*=Alkyl (vorzugsweise C₁- bis C₈-Alkyl) oder Aryl, besonders C₆-C₁₀-Aryl (vorzugsweise Phenyl) und die Derivate der Phosphinigsäure der allgemeinen Formel (III) 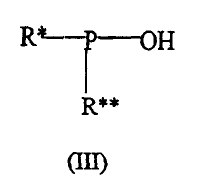 mit R** unabhängig von R* - mit R*, R** = Alkyl (vorzugsweise C₁- bis C₂₀-Alkyl) oder Aryl, besonders C₆-C₁₀-Aryl (vorzugsweise Phenyl).

Auch Derivate der bisher beschriebenen sauerstoffhaltigen Phosphorver-bindungen, welche mindestens ein die Oxidationszahl +3 aufweisendes Phosphoratom enthalten, bei denen ein oder mehrere O-Atome durch S oder NR* (R* hat dabei die gleiche Bedeutung wie vorstehend aufgeführt) ersetzt sind, sind geeignet.

Neben ein oder mehreren chemischen Verbindungen, welche mindestens ein die Oxidationszahl +3 aufweisendes Phosphoratom enthalten, ist in der erfindungsgemäßen Inhibitorkomposition u.a. als weitere Komponente (weitere Inhibitorkomponente) noch wenigstens ein Nitroxyl-Radikal(derivat) als Komponente a) enthalten.

Bevorzugt wird 4-Hydroxy-1-oxyl-2,2,6,6-tetramethylpiperidin als Nitroxyl-Radikal(derivat) eingesetzt.

Als geeignete Nitroxyl-Radikal(derivate) (auch als N-Oxyl-Radikale bezeichnet) kommen erfindungsgemäß prinzipiell alle Verbindungen in Betracht, die wenigstens eine >N-O• - Gruppe aufweisen. Die Nitroxyl-Radikal(derivate) können auch in situ aus anderen Verbindungen erzeugt werden, z.B. durch H-Abstraktion aus Hydroxylaminen oder durch Addition von C-Radikalen an Nitrone. Sie können aber auch aus aromatischen Aminen, die sich vom Anilin oder Phenylendiamin ableiten, in situ erzeugt werden. Als erfindungsgemäß geeignete Nitroxyl-Radikal(derivate) kommen vor allem diejenigen in Betracht, die sich von einem sekundären Amin ableiten, welches keine Wasserstoffatome an den α-C-Atomen trägt (d.h., die N-Oxyl-Gruppen leiten sich von entsprechenden sekundären Aminogruppen ab).

Solche geeigneten, sich von einem sekundären Amin ableitenden, stabilen Nitroxyl-Radikal(derivate) sind z.B. jene der allgemeinen Formel (IV) 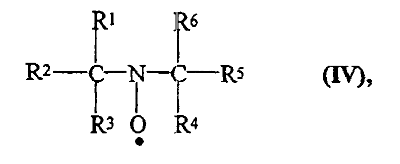 mit
- R¹, R², R⁵ und R⁶=: dieselben oder verschiedene gerad- oder verzweigtkettige, gegebenenfalls substituierte Alkylgruppen und
- R³ und R⁴ =: dieselben oder verschiedene gerad- oder verzweigtkettige, gegebenenfalls substituierte Alkylgruppen oder
- R³CNCR⁴ =: eine, gegebenenfalls substituierte, zyklische Struktur.

Beispiele für geeignete Verbindungen sind jene stabilen Nitroxyl-Radikal-(derivate) der allgemeinen Formel (IV), bei welchen R¹, R², R⁵ und R⁶ für (gleiche oder verschiedene) C₁- bis C₄-Alkylgruppen wie Methyl-, Ethyl-, n-Propyl-, iso-Propyl-, n-Butyl-, iso-Butyl- oder tert.-Butyl-, lineares oder verzweigtes Pentyl-, Phenyl- oder substituierte Gruppen hiervon und R³ und R⁴ für (gleiche oder verschiedene) C₁- bis C₄-Alkylgruppen wie Methyl-, Ethyl-, n-Propyl-, iso-Propyl-, n-Butyl-, iso-Butyl- oder tert.-Butyl-, lineares oder verzweigtes Pentyl-, substituierte Gruppen hiervon oder gemeinsam mit CNC für die zyklische Struktur 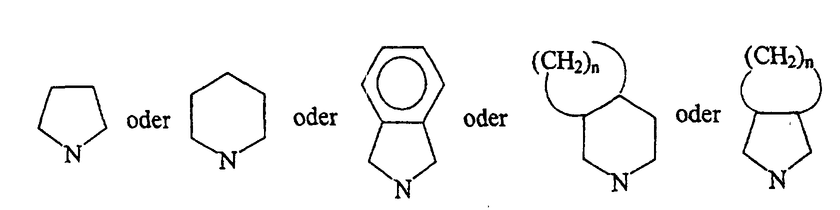 mit n gleich einer ganzen Zahl von 1 bis 10 (häufig 1 bis 6), einschließlich substituierter derartiger zyklischer Strukturen, stehen. Als beispielhafte Vertreter seien 2,2,6,6-Tetramethyl-1-oxyl-piperidin, 2,2,5,5-Tetramethyl-1-oxyl-pyrrolidin und 4-Oxo-2,2,6,6-tetramethyl-1-oxyl-piperidin genannt.

Die N-Oxyl-Radikal(derivate) der allgemeinen Form (IV) lassen sich aus den entsprechenden sekundären Aminen durch Oxidation, z.B. mit Wasserstoffper-oxid, herstellen. In der Regel sind sie als Reinsubstanz darstellbar.

Zu den erfindungsgemäß geeigneten Nitroxyl-Radikal(derivaten) zählen insbe-sondere Piperidin- oder Pyrrolidin-N-Oxyle und Di-N-Oxyle der nachstehenden allgemeinen Formeln (V) bis (XII): 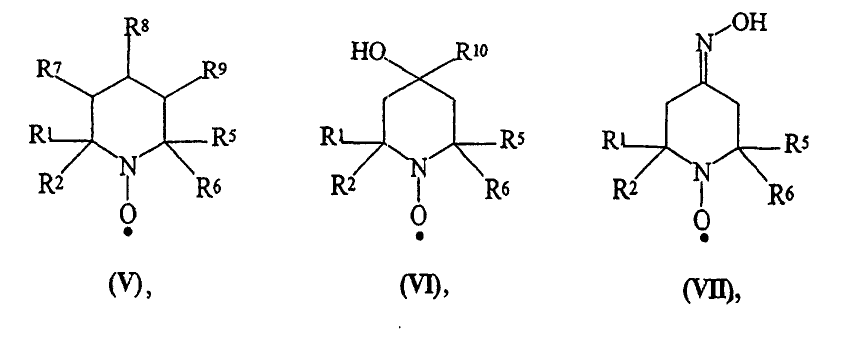 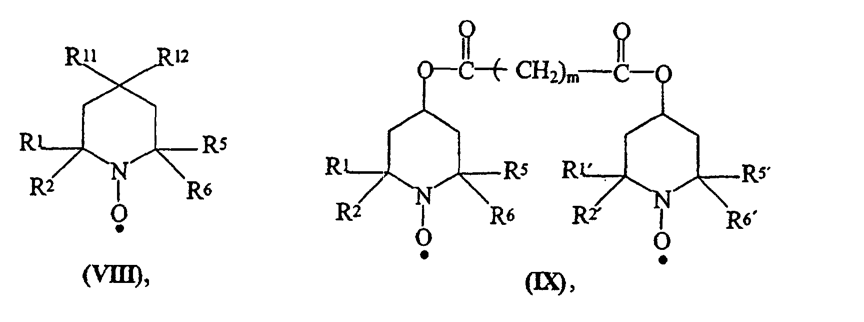 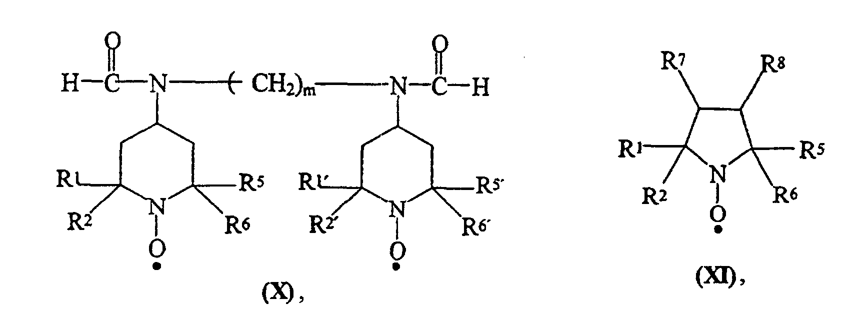 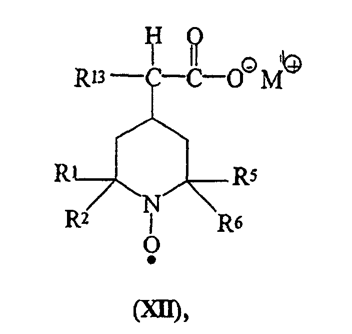 mit
m = 2 bis 10.
-R⁷, -R⁸, -R⁹ = unabhängig voneinander

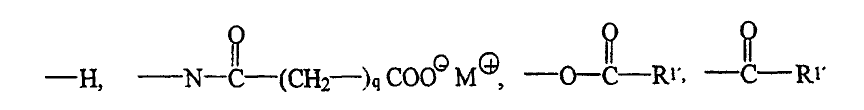

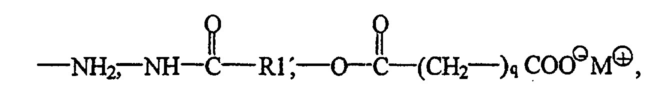

―COO^{⊖}M^{⊕},―SO₃^{⊖}M^{⊕},―PO₃^{⊖}M^{⊕},

―O―PO² ₃^{⊖} M₂^{⊕},―O―SO₃^{⊖}M^{⊕},―OH,

―O―(CH₂―CH₂―O―)_{q}H

oder 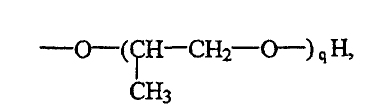
- M^{⊕} =: ein Wasserstoff- oder ein Alkalimetallion,
- q =: eine ganze Zahl von 1 bis 10,
- R^{1'}, R^{2'}, R^{5'}, R^{6'}=: unabhängig voneinander und unabhängig von R¹, R², R⁵, R⁶ dieselben Gruppen wie R¹,
- R¹⁰ =: C₁- bis C₄-Alkyl, -CH=CH₂, -C≡CH, -CN, 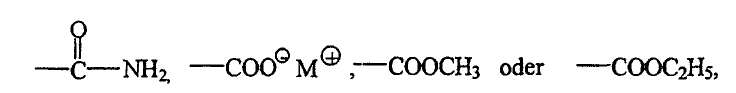
- R¹¹ =: ein organischer Rest, der wenigstens eine primäre, sekundäre (z.B.-NHR¹) oder tertiäre Aminogruppe (z.B. -NR¹R²) oder wenigstens eine Ammoniumgruppe - N^{⊕}R¹⁴R¹⁵R¹⁶X^{⊖} aufweist, mit X^{⊖} = F^{⊖}, Cl^{⊖}, Br^{⊖}, HSO₄^{⊖}, SO₄^{2⊖}, H₂PO₄^{⊖}, HPO₄^{2⊖} oder PO₄^{3⊖} und R¹⁴, R¹⁵, R¹⁶ voneinander unabhängige organische Reste (z.B. unabhängig voneinander und unabhängig von R¹ dieselben Gruppen wie R¹),
- R¹² =: unabhängig von R¹¹ dieselben Gruppen wie R¹¹ oder -H, -OH, C₁- bis C₄-Alkyl, -COO^{⊖}M^{⊕}, -C≡CH, 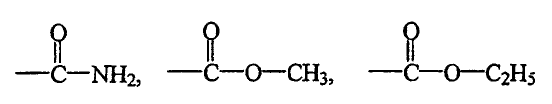 oder hydroxysubstituiertes C₁- bis C₄-Alkyl (z.B. hydroxyethyl oder hydroxypropyl) oder
- R¹¹, R¹² =: gemeinsam den Sauerstoff einer Carbonylgruppe und
- R¹³: 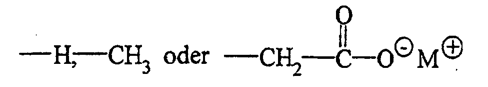
Vorzugsweise ist R¹ = R² = R⁵ = R⁶ = R^{1'} = R^{2'} = R^{5'} = R^{6'} = -CH₃.

Als beispielhafte Vertreter erfindungsgemäß geeigneter N-Oxyl-Radikal(derivate) seien 1-Oxyl-2,2,6,6-tetramethylpiperidin, 1-Oxyl-2,2,6,6-tetramethylpiperidin-4-ol, 1-Oxyl-2,2,6,6-tetramethyl-4-methoxypiperidin, 1-Oxyl-2,2,6,6-tetramethyl-4-ethoxypiperidin, 1-Oxyl-2,2,6,6-tetramethyl-4-trimethylsiloxypiperidin, 1-Oxyl-2,2,6,6-tetramethylpiperidin-4-on, 1-Oxyl-2,2,6,6-tetramethylpiperidin-4-yl-acetat, 1-Oxyl-2,2,6,6-tetramethylpiperidin-4-yl-2-ethylhexanoat, 1-Oxyl-2,2,6,6-tetramethylpiperidin-4-yl-stearat, 1-Oxyl-2,2,6,6-tetramethylpiperidin-4-yl-benzoat, 1-Oxyl-2,2,6,6-tetramethylpiperidin-4-yl-(4-tertbutyl)benzoat, Bis(1-oxyl-2,2,6,6-tetramethylpiperidin-4-yl)-succinat, Bis(1-oxyl-2,2,6,6-tetramethyl-piperidin-4-yl)-adipat, Bis(1-oxyl-2,2,6,6-tetramethylpiperidin-4-yl)-sebacat, Bis(1-oxyl-2,2,6,6-tetramethylpiperidin-4-yl)-n-butylmalonat, Bis(1-oxyl-2,2,-6,6-tetramethylpiperidin-4-yl)-phthalat, Bis(1-oxyl-2,2,6,6-tetramethylpiperidin-4-yl)-isophthalat, Bis(1-oxyl-2,2,6,6-tetramethylpiperidin-4-yl)-terephthalat, Bis(1-oxyl-2,2,6,6-tetramethylpiperidin-4-yl)-hexahydroterephthalat, N,N'-Bis-(1-oxyl-2,2,6,6-tetramethylpiperidin-4-yl)-adipinamid, N-(1-Oxyl-2,2,6,6-tetra-methylpiperidin-4-yl)-caprolactam, N-(1-Oxyl-2,2,6,6-tetra-methylpiperidin-4-yl)-dodecylsuccinimid, 2,4,6-Tris-[N-butyl-N-(1-oxyl-2,2,6,6-tetramethyl-piperidin-4-yl]-s-triazin, N,N'-Bis(1-oxyl-2,2,6,6-tetramethylpiperidin-4-yl)-N,N'-bis-formyl-1,6-diaminohexan, 4,4'-Ethylen-bis(1-oxyl-2,2,6,6-tetramethyl-piperazin-3-on) und Tris-(2,2,6,6-tetramethyl-1-oxyl-piperidin-4-yl)phosphit sowie zusätzlich noch 1-Oxyl-2,2,6,6-tetramethyl-1,2,3,6-tetrahydropyridin genannt.

Weitere geeignete beispielhafte Vertreter (bei den entsprechenden Strukturformeln sind H-Atome nicht eingezeichnet) sind: 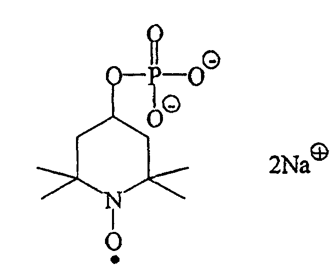 Sunamoto, Junzo; Akiyoshi, Kuzunari, Kihara, Tetsuji; Endo, Masayuki, BCS JA 8, Bull. Chem. Soc. Jpn., EN, 65, 4, 1992, S. 1041 - 1046; 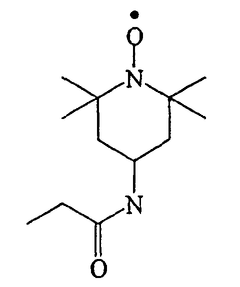 Beilstein Registry Number 6926369 (C₁₁H₂₂N₃O₂); 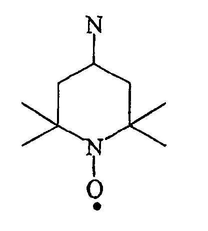 Beilstein Registry Number 6498805 (4-Amino-2,2,6,6-tetramethyl-1-oxylpiperidin); 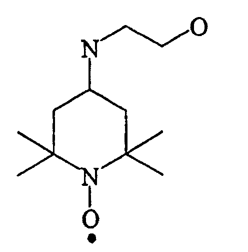 Beilstein Registry Number 6800244 (C₁₁H₂₃N₂O₂); 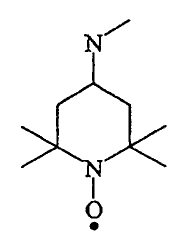 Beilstein Registry Number 5730772 (N-Methyl-4-amino)-2,2,6,6-tetramethyl-1-oxyl-piperidin; 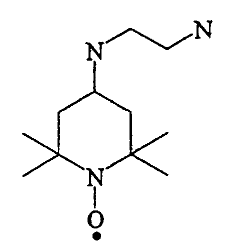 Beilstein Registry Number 5507538 (2,2,6,6-Tetramethyl-4-(2-aminoethylamino)-1-oxyl-piperidin); 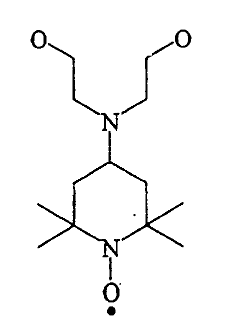 Beilstein Registry Number 4417950 (4<Bis(2-hydroxyethyl)>-amino-2,2,6,6-tetramethyl-1-oxyl-piperidin); 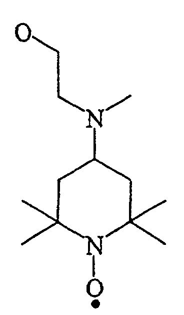 Beilstein Registry Number 4396625 (C₁₂H₂₅N₂O₂); 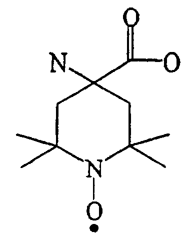 Beilstein Registry Number 4139900 (4-Amino-2,2,6,6-tetramethyl-4-carboxy-1-oxyl-piperidin); 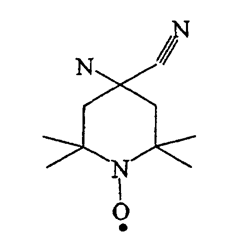 Beilstein Registry Number 4137088 (4-Amino-4-cyano-2,2,6,6-tetramethyl-1-oxyl-piperidin); 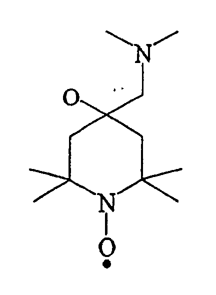 Beilstein Registry Number 3942714 (C₁₂H₂₅N₂O₂); 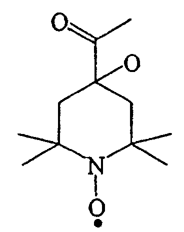 Beilstein Registry Number 1468515 (2,2,6,6-Tetramethyl-4-hydroxy-4-acetyl-1-oxyl-piperidin); 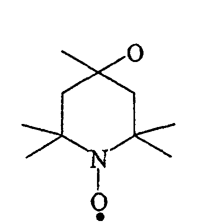 Beilstein Registry Number 1423410 (2,2,4,6,6-Pentamethyl-4-hydroxy-1-oxyl-piperidin); 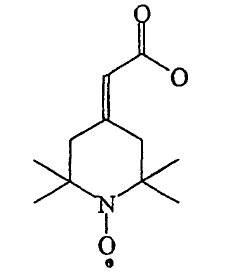 Beilstein Registry Number 6205316 (4-Carboxymethylen-2,2,6,6-tetramethyl-1-oxyl-piperidin); 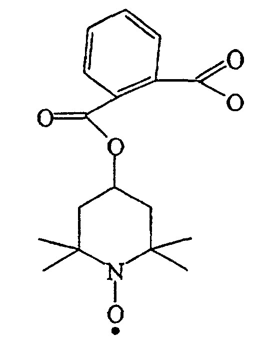 Beilstein Registry Number 1395538 (4-<2-Carboxylbenzoyloxy>-2,2,6,6-tetramethyl-1-oxyl-piperidin); 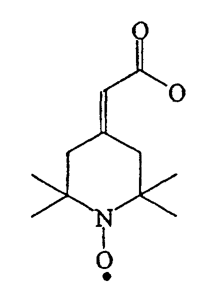 Beilstein Registry Number 3546230 (4-Carboxymethyl-2,2,6,6-tetramethyl-1-oxyl-piperidin); 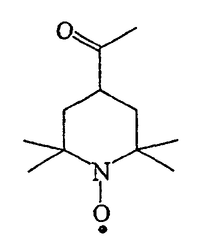 Beilstein Registry Number 3949026 (4-Carboxyl-2,2,6,6-tetramethyl-1-oxyl-piperidin); 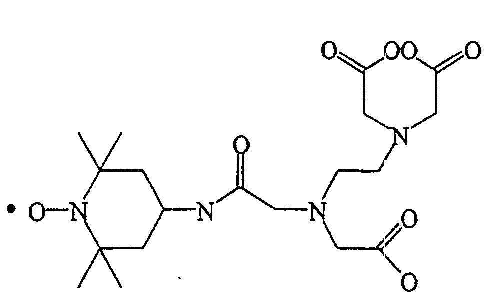 Beilstein Registry Number 4611003 Ethylendiamintetraessigsäuremono(1-oxy-2,2,6,6-tetramethylpiperidinyl-4-amid); 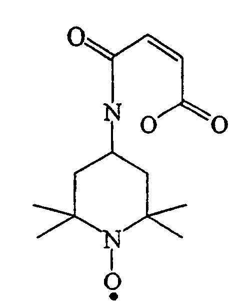 Beilstein Registry Number 5961636 (C₁₃H₂₁N₂O₄) 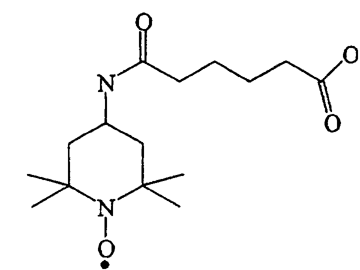 Beilstein Registry Number 5592232 (C₁₅H₂₇N₂O₄); 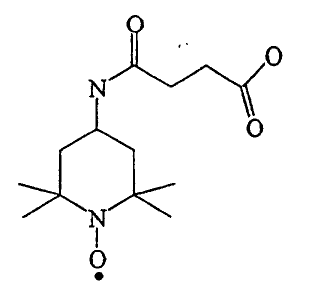 Beilstein Registry Number 5080576 (Bemsteinsäure-N-(2,2,6,6-tetramethyl-1-oxyl-4-piperidinyl)-monoamid); 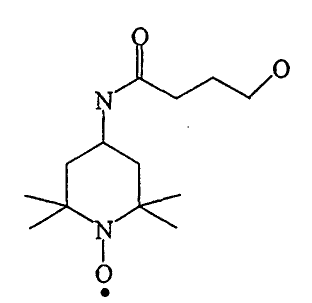 Beilstein Registry Number 5051814 (4- (4-Hydroxybutanoylamino)-2,2,6,6-tetramethyl-1-oxyl-piperidin); 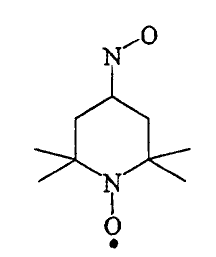 Beilstein Registry Number 4677496 (2,2,6,6-Tetramethyl-4-oximino-1-oxyl-piperidin); 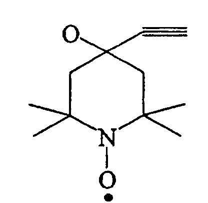 Beilstein Registry Number 1451068 (C₁₁H₁₈NO₂); 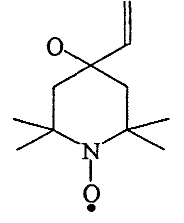 Beilstein Registry Number 1451075 (C₁₁H₂₀NO₂); 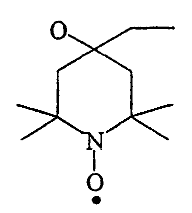 Beilstein Registry Number 1423698 (4-Ethyl-4-hydroxy-2,2,6,6-tetramethyl-1-oxyl-piperidin); 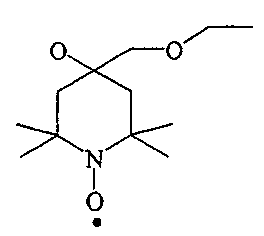 Beilstein Registry Number 5509793 (4-Ethoxymethyl-4-hydroxy-2,2,6,6-tetramethyl-1-oxyl-piperidin); 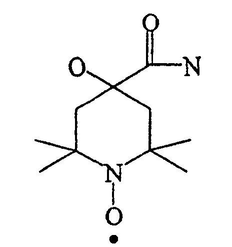 Beilstein Registry Number 3960373 (C₁₀H₁₉N₂O₃); 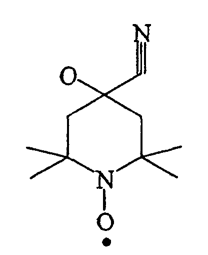 Beilstein Registry Number (C₁₀H₁₇N₂O₂); 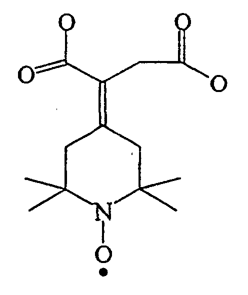 Beilstein Registry Number 3985130 (2,2,6,6-Tetramethyl-1-oxyl-4-piperidyliden)-bernsteinsäure);

Selbstverständlich können erfindungsgemäß auch Gemische von N-Oxyl-Radikal(derivaten) eingesetzt werden.

Die Inhibitorkomposition enthält als weitere Komponente - Komponente b) - noch wenigstens ein Phenol(derivat) (als Phenolderivat kommt auch Phenol als solches in Frage). Als Phenolderivat wird bevorzugt ein Phenol oder Kresol, bei dem ein oder mehrere Wasserstoffatome durch Tert.-Butyl-Gruppen substituiert sind, insbesondere 2,6-Di-tert.-butylkresol, oder Hydrochinone, insbesondere Hydrochinonmonomethylether, eingesetzt. Außerdem kommen Phenole oder Kresole in Frage, bei denen ein oder mehrere Wasserstoffatome durch i-Propyl-Gruppen substituiert sind, wie Thymol oder Carvacrol. Außerdem eignen sich Brenzkatechin und dessen Derivate sowie Resorcin und dessen Derivate. Wie vorstehend beschrieben, eignen sich als zweiwertige Phenole auch Hydrochinon und dessen Derivate, insbesondere Ether des Hydrochinons wie Hydrochinonmonomethylether. Naphthole, wie α-Naphthol und Vitamin E kommen ebenfalls in Frage. Außerdem eignen sich auch Phenole, die über Alkylbrücken miteinander verbunden sind, wie Bisphenol A. Allgemein eignen sich als Phenolderivate Verbindungen, die ein Oxylradikal bilden können, das an ein aromatisches System gebunden ist. Dies geschieht durch homolytische Spaltung einer Sauerstoff-Wasserstoffbindung, wobei das Sauerstoffatom an ein aromatisches System gebunden ist. Besonders bevorzugte Phenolderivate werden durch die folgende allgemeine Strukturformel (XIII) wiedergegeben:

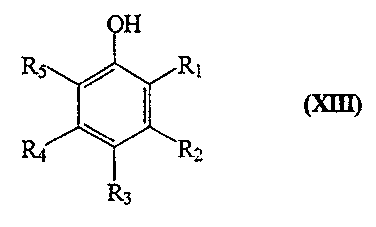

Dabei sind R₁, R₂, R₃, R₄, und R₅ gleich oder verschieden und bedeuten H, Halogen, C₁ bis C₂₀ - Alkyl oder Aryl, SO₃H, SO₃⁻M⁺, OH, SH, O-Alkyl, O-Aryl, S-Alkyl, S-Aryl, NH-Akyl, NH-Aryl, NO, NO₂, NH-OH, NH₂, COOH, CN, O-CO-R6, O-CO-O-R₆, NH-CO-R₆, CN(-OH)-(R₆), CO-O-R₆ oder CO-NH-R₆, wobei R₆ = H, Halogen, C₁ bis C₂₀ - Alkyl oder Aryl, SO₃H, SO₃⁻M⁺, OH, SH, O-Alkyl, O-Aryl, S-Alkyl, S-Aryl, NH-Akyl, NH-Aryl, NO, NO₂, NH-OH, NH₂, COOH oder CN.

Die Inhibitorkomposition enthaltend ein oder mehrere chemische Verbindungen, welche mindestens ein die Oxidationszahl +3 aufweisendes Phosphoratom enthalten, sowie ein oder mehrere Nitroxyl-Radikal(derivate) und ein oder mehrere Phenol(derivate), weist eine besonders gute stabilisierende Wirkung auf. Bezüglich der Wirkung als Inhibitor bei der radikalischen Polymerisation weist die erfindungsgemäße Inhibitorkomposition synergistische Eigenschaften auf - Spezies der drei verschiedenen Inhibitorkomponenten verstärken sich gegenseitig in ihrer inhibierenden Wirkung. Demgemäß ist die inhibierende Wirkung der erfindungsgemäßen Kombination besser als die inhibierende Wirkung, die zum Beispiel beim alleinigen Vorliegen der einzelnen Komponenten vorhanden wäre, oder als die Addition der Einzelwirkungen.

Günstig ist, daß die erfindungsgemäße Inhibitorkomposition ihre Wirksamkeit auch im Beisein von molekularem Sauerstoff entfaltet.

Im folgenden soll auf besonders geeignete Inhibitorkompositionen eingegangen werden. Dabei sind Komponenten, die in diesen Inhibitorkompositionen enthalten sind, in der nachstehenden Tabelle aufgeführt - die Kombination folgender Komponenten erweist sich als besonders günstig:

| *Komponente a)* | *Komponente c)* | *Komponente b)* |
|---|---|---|
| 4-Hydroxy-1-oxyl-2,2,6,6-tetramethylpiperidin | P(OH)₃ | 4-Tert.-butylphenol |
| 4-Hydroxy-1-oxyl-2,2,6,6-tetramethylpiperidin | P(OMethyl)₃ | 4-Tert.-butylphenol |
| 4-Hydroxy-1-oxyl-2,2,6,6-tetramethylpiperidin | P(OEthyt)₃ | 4-Tert.-butylphenol |
| 4-Hydroxy-1-oxyl-2,2,6,6-tetramethylpiperidin | P(Oiso-Propyl)₃ | 4-Tert.-butylphenol |
| 4-Hydroxy-1-oxyl-2,2,6,6-tetramethylpiperidin | P(OPropyl)₃ | 4-Tert.-butylphenol |
| 4-Hydroxy-1-oxyl-2,2,6,6-tetramethylpiperidin | P(On-Butyl)₃ | 4-Tert.-butylphenol |
| 4-Hydroxy-1-oxyl-2,2,6,6-tetramethylpiperidin | P(Osec-Butyl)₃ | 4-Tert.-butylphenol |
| 4-Hydroxy-1-oxyl-2,2,6,6-tetramethylpiperidin | P(Otert.-Butyl)₃ | 4-Tert.-butylphenol |
| 4-Hydroxy-1-oxyl-2,2,6,6-tetramethylpiperidin | P(OPhenyl)₃ | 4-Tert.-butylphenol |
| 4-Hydroxy-1-oxyl-2,2,6,6-tetramethylpiperidin | P(OH)(OMethyl)₂ | 4-Tert.-butylphenol |
| 4-Hydroxy-1-oxyl-2,2,6,6-tetramethylpiperidin | P(OH)(OEthyl)₂ | 4-Tert.-butylphenol |
| 4-Hydroxy-1-oxyl-2,2,6,6-tetramethylpiperidin | P(OH)(Oiso-Propyl)₂ | 4-Tert.-butylphenol |
| 4-Hydroxy-1-oxyl-2,2,6,6-tetramethylpiperidin | P(OH)(OPropyl)₂ | 4-Tert.-butylphenol |
| 4-Hydroxy-1-oxyl-2,2,6,6-tetramethylpiperidin | P(OH)(On-Butyl)₂ | 4-Tert.-butylphenol |
| 4-Hydroxy-1-oxyl-2,2,6,6-tetramethylpiperidin | P(OH)(Osec-Butyl)₂ | 4-Tert.-butylphenol |
| 4-Hydroxy-1-oxyl-2,2,6,6-tetramethylpiperidin | P(OH)(Otert.-Butyl)₂ | 4-Tert.-butylphenol |
| 4-Hydroxy-1-oxyl-2,2,6,6-tetramethylpiperidin | P(OH)(OPhenyl)₂ | 4-Tert.-butylphenol |
| 4-Hydroxy-1-oxyl-2,2,6,6-tetramethylpiperidin | P(OH)₂(OMethyl) | 4-Tert.-butylphenol |
| 4-Hydroxy-1-oxyl-2,2,6,6-tetramethylpiperidin | P(OH)₂(OEthyl) | 4-Tert.-butylphenol |
| 4-Hydroxy-1-oxyl-2,2,6,6-tetramethylpiperidin | P(OH)₂(Oiso-Propyl) | 4-Tert.-butylphenol |
| 4-Hydroxy-1-oxyl-2,2,6,6-tetramethylpiperidin | P(OH)₂(OPropyl) | 4-Tert.-butylphenol |
| 4-Hydroxy-1-oxyl-2,2,6,6-tetramethylpiperidin | P(OH)₂(On-Butyl) | 4-Tert.-butylphenol |
| 4-Hydroxy-1-oxyl-2,2,6,6-tetramethylpiperidin | P(OH)₂(Osec-Butyl) | 4-Tert.-butylphenol |
| 4-Hydroxy-1-oxyl-2,2,6,6-tetramethylpiperidin | P(OH)₂(Otert.-Butyl) | 4-Tert.-butylphenol |
| 4-Hydroxy-1-oxyl-2,2,6,6-tetramethylpiperidin | P(OH)₂(OPhenyl) | 4-Tert.-butylphenol |
| 4-Hydroxy-1-oxyl-2,2,6,6-tetramethylpiperidin | (Methyl)P(OMethyl)₂ | 4-Tert.-butylphenol |
| 4-Hydroxy-1-oxyl-2,2,6,6-tetramethylpiperidin | (Ethyl)P(OEthyl)₂ | 4-Tert.-butylphenol |
| 4-Hydroxy-1-oxyl-2,2,6,6-tetramethylpiperidin | (iso-Propyl)P(Oiso-Propyl)₂ | 4-Tert.-butylphenol |
| 4-Hydroxy-1-oxyl-2,2,6,6-tetramethylpiperidin | (Propyl)P(OPropyl)₂ | 4-Tert.-butylphenol |
| 4-Hydroxy-1-oxyl-2,2,6,6-tetramethylpiperidin | (n-Butyl)P(On-Butyl)₂ | 4-Tert.-butylphenol |
| 4-Hydroxy-1-oxyl-2,2,6,6-tetramethylpiperidin | (sec-Butyl)P(Osec-Butyl)₂ | 4-Tert.-butylphenol |
| 4-Hydroxy-1-oxyl-2,2,6,6-tetramethylpiperidin | (tert.-Butyl)P(Otert.-Butyl)₂ | 4-Tert.-butylphenol |
| 4-Hydroxy-1-oxyl-2,2,6,6-tetramethylpiperidin | (Penyl)P(OPhenyl)₂ | 4-Tert.-butylphenol |
| 4-Hydroxy-1-oxyl-2,2,6,6-tetramethylpiperidin | RP(OH)₂ | 4-Tert.-butylphenol |
| 4-Hydroxy-1-oxyl-2,2,6,6-tetramethylpiperidin | RP(OH)(OR) | 4-Tert.-butylphenol |
| mit R = Me, Et, iso-Pr, n-Pr, n-Bu, sec-Bu, tert-Bu, Phenyl | | |

| *Komponente a)* | *Komponente c)* | *Komponente b)* |
|---|---|---|
| 4-Hydroxy-1-oxyl-2,2,6,6-tetramethylpiperidin | P(OH)₃ | 2,4-Di-tert.-butylphenol |
| 4-Hydroxy-1-oxyl-2,2,6,6-tetramethylpiperidin | P(OMethyl)₃ | 2,4-Di-tert.-butylphenol |
| 4-Hydroxy-1-oxyl-2,2,6,6-tetramethylpiperidin | P(OEthyl)₃ | 2,4-Di-tert.-butylphenol |
| 4-Hydroxy-1-oxyl-2,2,6,6-tetramethylpiperidin | P(Oiso-Propyl)₃ | 2,4-Di-tert.-butylphenol |
| 4-Hydroxy-1-oxyl-2,2,6,6-tetramethylpiperidin | P(OPropyl)₃ | 2,4-Di-tert.-butylphenol |
| 4-Hydroxy-1-oxyl-2,2,6,6-tetramethylpiperidin | P(On-Butyl)₃ | 2,4-Di-tert.-butylphenol |
| 4-Hydroxy-1-oxyl-2,2,6,6-tetramethylpiperidin | P(Osec-Butyl)₃ | 2,4-Di-tert.-butylphenol |
| 4-Hydroxy-1-oxyl-2,2,6,6-tetramethylpiperidin | P(Otert.-Butyl)₃ | 2,4-Di-tert.-butylplienol |
| 4-Hydroxy-1-oxyl-2,2,6,6-tetramethylpiperidin | P(OPhenyl)₃ | 2,4-Di-tert.-butylphenol |
| 4-Hydroxy-1-oxyl-2,2,6,6-tetramethylpiperidin | P(OH)(OMethyl)₂ | 2,4-Di-tert.-butylphenol |
| 4-Hydroxy-1-oxyl-2,2,6,6-tetramethylpiperidin | P(OH)(OEthyl)₂ | 2,4-Di-tert.-butylphenol |
| 4-Hydroxy-1-oxyl-2,2,6,6-tetramethylpiperidin | P(OH)(Oiso-Propyl)₂ | 2,4-Di-tert.-butylphenol |
| 4-Hydroxy-1-oxyl-2,2,6,6-tetramethylpiperidin | P(OH)(OPropyl)₂ | 2,4-Di-tert.-butylphenol |
| 4-Hydroxy-1-oxyl-2,2,6,6-tetramethylpiperidin | P(OH)(On-Butyl)₂ | 2,4-Di-tert.-butylphenol |
| 4-Hydroxy-1-oxyl-2,2,6,6-tetramethylpiperidin | P(OH)(Osec-Butyl)₂ | 2,4-Di-tert.-butylphenol |
| 4-Hydroxy-1-oxyl-2,2,6,6-tetramethylpiperidin | P(OH)(Otert.-Butyl)₂ | 2,4-Di-tert.-butylphenol |
| 4-Hydroxy-1-oxyl-2,2,6,6-tetramethylpiperidin | P(OH)(OPhenyl)₂ | 2,4-Di-tert.-butylphenol |
| 4-Hydroxy-1-oxyl-2,2,6,6-tetramethylpiperidin | P(OH)₂(OMethyl) | 2,4-Di-tert.-butylphenol |
| 4-Hydroxy-1-oxyl-2,2,6,6-tetramethylpiperidin | P(OH)₂(OEthyl) | 2,4-Di-tert.-butylphenol |
| 4-Hydroxy-1-oxyl-2,2,6,6-tetramethylpiperidin | P(OH)₂(Oiso-Propyl) | 2,4-Di-tert.-butylphenol |
| 4-Hydroxy-1-oxyl-2,2,6,6-tetramethylpiperidin | P(OH)₂(OPropyl) | 2,4-Di-tert.-butylphenol |
| 4-Hydroxy-1-oxyl-2,2,6,6-tetramethylpiperidin | P(OH)₂(On-Butyl) | 2,4-Di-tert.-butylphenol |
| 4-Hydroxy-1-oxyl-2,2,6,6-tetramethylpiperidin | P(OH)₂(Osec-Butyl) | 2,4-Di-tert.-butylphenol |
| 4-Hydroxy-1-oxyl-2,2,6,6-tetramethylpiperidin | P(OH)₂(Otert.-Butyl) | 2,4-Di-tert.-butylphenol |
| 4-Hydroxy-1-oxyl-2,2,6,6-tetramethylpiperidin | P(OH)₂(OPhenyl) | 2,4-Di-tert.-butylphenol |
| 4-Hydroxy-1-oxyl-2,2,6,6-tetramethylpiperidin | (Methyl)P(OMethyl)₂ | 2,4-Di-tert.-butylphenol |
| 4-Hydroxy-1-oxyl-2,2,6,6-tetramethylpiperidin | (Ethyl)P(OEthyl)₂ | 2,4-Di-tert.-butylphenol |
| 4-Hydroxy-1-oxyl-2,2,6,6-tetramethylpiperidin | (iso-Propyl)P(Oiso-Propyl)₂ | 2,4-Di-tert.-butylphenol |
| 4-Hydroxy-1-oxyl-2,2,6,6-tetramethylpiperidin | (Propyl)P(OPropyl)₂ | 2,4-Di-tert.-butylphenol |
| 4-Hydroxy-1-oxyl-2,2,6,6-tetramethylpiperidin | (n-Butyl)P(On-Butyl)₂ | 2,4-Di-tert.-butylphenol |
| 4-Hydroxy-1-oxyl-2,2,6,6-tetramethylpiperidin | (sec-Butyl)P(Osec-Butyl)₂ | 2,4-Di-tert.-butylphenol |
| 4-Hydroxy-1-oxyl-2,2,6,6-tetramethylpiperidin | (tert.-Butyl)P(Otert.-Butyl)₂ | 2,4-Di-tert.-butylphenol |
| 4-Hydroxy-1-oxyl-2,2,6,6-tetramethylpiperidin | (Penyl)P(OPhenyl)₂ | 2,4-Di-tert.-butylphenol |
| 4-Hydroxy-1-oxyl-2,2,6,6-tetramethylpiperidin | RP(OH)₂ | 2,4-Di-tert.-butylphenol |
| 4-Hydroxy-1-oxyl-2,2,6,6-tetramethylpiperidin | RP(OHXOR) | 2,4-Di-tert.-butylphenol |
| mit R = Me, Et, iso-Pr, n-Pr, n-Bu, sec-Bu, tert-Bu, Phenyl | | |

| *Komponente a)* | *Komponente c)* | *Komponente b)* |
|---|---|---|
| 4-Hydroxy-1-oxyl-2,2,6,6-tetramethylpiperidin | P(OH)₃ | Hydrochinonmonomethylether |
| 4-Hydroxy-1-oxyl-2,2,6,6-tetramethylpiperidin | P(OMethyl)₃ | Hydrochinonmonomethylether |
| 4-Hydroxy-1-oxyl-2,2,6,6-tetramethylpiperidin | P(OEthyl)₃ | Hydrochinonmonomethylether |
| 4-Hydroxy-1-oxyl-2,2,6,6-tetramethylpiperidin | P(Oiso-Propyl)₃ | Hydrochinonmonomethylether |
| 4-Hydroxy-1-oxyl-2,2,6,6-tetramethylpiperidin | P(OPropyl)₃ | Hydrochinonmonomethylether |
| 4-Hydroxy-1-oxyl-2,2,6,6-tetramethylpiperidin | P(On-Butyl)₃ | Hydrochinonmonomethylether |
| 4-Hydroxy-1-oxyl-2,2,6,6-tetramethylpiperidin | P(Osec-Butyl)₃ | Hydrochinonmonomethylether |
| 4-Hydroxy-1-oxyl-2,2,6,6-tetramethylpiperidin | P(Otert.-Butyl)₃ | Hydrochinonmonomethylether |
| 4-Hydroxy-1-oxyl-2,2,6,6-tetramethylpiperidin | P(OPhenyl)₃ | Hydrochinonmonomethylether |
| 4-Hydroxy-1-oxyl-2,2,6,6-tetramethylpiperidin | P(OH)(OMethyl)₂ | Hydrochinonmonomethylether |
| 4-Hydroxy-1-oxyl-2,2,6,6-tetramethylpiperidin | P(OH)(OEthyl)₂ | Hydrochinonmonomethylether |
| 4-Hydroxy-1-oxyl-2,2,6,6-tetramethylpiperidin | P(OH)(Oiso-Propyl)₂ | Hydrochinonmonomethylether |
| 4-Hydroxy-1-oxyl-2,2,6,6-tetramethylpiperidin | P(OH)(OPropyl)₂ | Hydrochinonmonomethylether |
| 4-Hydroxy-1-oxyl-2,2,6,6-tetramethylpiperidin | P(OH)(On-Butyl)₂ | Hydrochinonmonomethylether |
| 4-Hydroxy-1-oxyl-2,2,6,6-tetramethylpiperidin | P(OH)(Osec-Butyl)₂ | Hydrochinonmonomethylether |
| 4-Hydroxy-1-oxyl-2,2,6,6-tetramethylpiperidin | P(OH)(Otert.-Butyl)₂ | Hydrochinonmonomethylether |
| 4-Hydroxy-1-oxyl-2,2,6,6-tetramethylpiperidin | P(OH)(OPhenyl)₂ | Hydrochinonmonomethylether |
| 4-Hydroxy-1-oxyl-2,2,6,6-tetramethylpiperidin | P(OH)₂(OMethyl) | Hydrochinonmonomethylether |
| 4-Hydroxy-1-oxyl-2,2,6,6-tetramethylpiperidin | P(OH)₂(OEthyl) | Hydrochinonmonomethylether |
| 4-Hydroxy-1-oxyl-2,2,6,6-tetramethylpiperidin | P(OH)₂(Oiso-Propyl) | Hydrochinonmonomethylether |
| 4-Hydroxy-1-oxyl-2,2,6,6-tetramethylpiperidin | P(OH)₂(OPropyl) | Hydrochinonmonomethylether |
| 4-Hydroxy-1-oxyl-2,2,6,6-tetramethylpiperidin | P(OH)₂(On-Butyl) | Hydrochinonmonomethylether |
| 4-Hydroxy-1-oxyl-2,2,6,6-tetramethylpiperidin | P(OH)₂(Osec-Butyl) | Hydrochinonmonomethylether |
| 4-Hydroxy-1-oxyl-2,2,6,6-tetramethylpiperidin | P(OH)₂(Otert.-Butyl) | Hydrochinonmonomethylether |
| 4-Hydroxy-1-oxyl-2,2,6,6-tetramethylpiperidin | P(OH)₂(OPhenyl) | Hydrochinonmonomethylether |
| 4-Hydroxy-1-oxyl-2,2,6,6-tetramethylpiperidin | (Methyl)P(OMethyl)₂ | Hydrochinonmonomethylether |
| 4-Hydroxy-1-oxyl-2,2,6,6-tetramethylpiperidin | (Ethyl)P(OEthyl)₂ | Hydrochinonmonomethylether |
| 4-Hydroxy-1-oxyl-2,2,6,6-tetramethylpiperidin | (iso-Propyl)P(Oiso-Propyl)₂ | Hydrochinonmonomethylether |
| 4-Hydroxy-1-oxyl-2,2,6,6-tetramethylpiperidin | (Propyl)P(OPropyl)₂ | Hydrochinonmonomethylether |
| 4-Hydroxy-1-oxyl-2,2,6,6-tetramethylpiperidin | (n-Butyl)P(On-Butyl)₂ | Hydrochinonmonomethylether |
| 4-Hydroxy-1-oxyl-2,2,6,6-tetramethylpiperidin | (sec-Butyl)P(Osec-Butyl)₂ | Hydrochinonmonomethylether |
| 4-Hydroxy-1-oxyl-2,2,6,6-tetramethylpiperidin | (tert.-Butyl)P(Otert.-Butyl)₂ | Hydrochinonmonomethylether |
| 4-Hydroxy-1-oxyl-2,2,6,6-tetramethylpiperidin | (Penyl)P(OPhenyl)₂ | Hydrochinonmonomethylether |
| 4-Hydroxy-1-oxyl-2,2,6,6-tetramethylpiperidin | RP(OH)₂ | Hydrochinonmonomethylether |
| 4-Hydroxy-1-oxyl-2,2,6,6-tetramethylpiperidin | RP(OH)(OR) | Hydrochinonmonomethylether |
| mit R = Me, Et, iso-Pr, n-Pr, n-Bu, sec-Bu, tert-Bu, Phenyl | | |

| *Komponente a)* | *Komponente c)* | *Komponente b)* |
|---|---|---|
| 4-Hydroxy-1-oxyl-2,2,6,6-tetramethylpiperidin | P(OH)₃ | Phenol |
| 4-Hydroxy-1-oxyl-2,2,6,6-tetramethylpiperidin | P(OMethyl)₃ | Phenol |
| 4-Hydroxy-1-oxyl-2,2,6,6-tetramethylpiperidin | P(OEthyl)₃ | Phenol |
| 4-Hydroxy-1-oxyl-2,2,6,6-tetramethylpiperidin | P(Oiso-Propyl)₃ | Phenol |
| 4-Hydroxy-1-oxyl-2,2,6,6-tetramethylpiperidin | P(OPropyl)₃ | Phenol |
| 4-Hydroxy-1-oxyl-2,2,6,6-tetramethylpiperidin | P(On-Butyl)₃ | Phenol |
| 4-Hydroxy-1-oxyl-2,2,6,6-tetramethylpiperidin | P(Osec-Butyl)₃ | Phenol |
| 4-Hydroxy-1-oxyl-2,2,6,6-tetramethylpiperidin | P(Otert.-Butyl)₃ | Phenol |
| 4-Hydroxy-1-oxyl-2,2,6,6-tetramethylpiperidin | P(OPhenyl)₃ | Phenol |
| 4-Hydroxy-1-oxyl-2,2,6,6-tetramethylpiperidin | P(OH)(OMethyl)₂ | Phenol |
| 4-Hydroxy-1-oxyl-2,2,6,6-tetramethylpiperidin | P(OH)(OEthyl)₂ | Phenol |
| 4-Hydroxy-1-oxyl-2,2,6,6-tetramethylpiperidin | P(OH)(Oiso-Propyl)₂ | Phenol |
| 4-Hydroxy-1-oxyl-2,2,6,6-tetramethylpiperidin | P(OH)(OPropyl)₂ | Phenol |
| 4-Hydroxy-1-oxyl-2,2,6,6-tetramethylpiperidin | P(OH)(On-Butyl)₂ | Phenol |
| 4-Hydroxy-1-oxyl-2,2,6,6-tetramethylpiperidin | P(OH)(Osec-Butyl)₂ | Phenol |
| 4-Hydroxy-1-oxyl-2,2,6,6-tetramethylpiperidin | P(OH)(Otert.-Butyl)₂ | Phenol |
| 4-Hydroxy-1-oxyl-2,2,6,6-tetramethylpiperidin | P(OH)(OPhenyl)₂ | Phenol |
| 4-Hydroxy-1-oxyl-2,2,6,6-tetramethylpiperidin | P(OH)₂(OMethyl) | Phenol |
| 4-Hydroxy-1-oxyl-2,2,6,6-tetramethylpiperidin | P(OH)₂(OEthyl) | Phenol |
| 4-Hydroxy-1-oxyl-2,2,6,6-tetramethylpiperidin | P(OH)₂(Oiso-Propyl) | Phenol |
| 4-Hydroxy-1-oxyl-2,2,6,6-tetramethylpiperidin | P(OH)₂(OPropyl) | Phenol |
| 4-Hydroxy-1-oxyl-2,2,6,6-tetramethylpiperidin | P(OH)₂(On-Butyl) | Phenol |
| 4-Hydroxy-1-oxyl-2,2,6,6-tetramethylpiperidin | P(OH)₂(Osec-Butyl) | Phenol |
| 4-Hydroxy-1-oxyl-2,2,6,6-tetramethylpiperidin | P(OH)₂(Otert.-Butyl) | Phenol |
| 4-Hydroxy-1-oxyl-2,2,6,6-tetramethylpiperidin | P(OH)₂(OPhenyl) | Phenol |
| 4-Hydroxy-1-oxyl-2,2,6,6-tetramethylpiperidin | (Methyl)P(OMethyl)₂ | Phenol |
| 4-Hydroxy-1-oxyl-2,2,6,6-tetramethylpiperidin | (Ethyl)P(OEthyl)₂ | Phenol |
| 4-Hydroxy-1-oxyl-2,2,6,6-tetramethylpiperidin | (iso-Propyl)P(Oiso-Propyl)₂ | Phenol |
| 4-Hydroxy-1-oxyl-2,2,6,6-tetramethylpiperidin | (Propyl)P(OPropyl)₂ | Phenol |
| 4-Hydroxy-1-oxyl-2,2,6,6-tetramethylpiperidin | (n-Butyl)P(On-Butyl)₂ | Phenol |
| 4-Hydroxy-1-oxyl-2,2,6,6-tetramethylpiperidin | (sec-Butyl)P(Osec-Butyl)₂ | Phenol |
| 4-Hydroxy-1-oxyl-2,2,6,6-tetramethylpiperidin | (tert.-Butyl)P(Otert.-Butyl)₂ | Phenol |
| 4-Hydroxy-1-oxyl-2,2,6,6-tetramethylpiperidin | (Penyl)P(OPhenyl)₂ | Phenol |
| 4-Hydroxy-1-oxyl-2,2,6,6-tetramethylpiperidin | RP(OH)₂ | Phenol |
| 4-Hydroxy-1-oxyl-2,2,6,6-tetramethylpiperidin | RP(OH)(OR) | Phenol |
| mit R = Me, Et, iso-Pr, n-Pr, n-Bu, sec-Bu, tert-Bu, Phenyl | | |

| *Komponente a)* | *Komponente c)* | *Komponente b)* |
|---|---|---|
| 4-Hydroxy-1-oxyl-2,2,6,6-tetramethylpiperidin | P(OH)₃ | Tert.-butylbrenzkatechin |
| 4-Hydroxy-1-oxyl-2,2,6,6-tetramethylpiperidin | P(OMethyl)₃ | Tert.-butylbrenzkatechin |
| 4-Hydroxy-1-oxyl-2,2,6,6-tetramethylpiperidin | P(OEthyl)₃ | Tert.-butylbrenzkatechin |
| 4-Hydroxy-1-oxyl-2,2,6,6-tetramethylpiperidin | P(Oiso-Propyl)₃ | Tert.-butylbrenzkatechin |
| 4-Hydroxy-1-oxyl-2,2,6,6-tetramethylpiperidin | P(OPropyl)₃ | Tert.-butylbrenzkatechin |
| 4-Hydroxy-1-oxyl-2,2,6,6-tetramethylpiperidin | P(On-Butyl)₃ | Tert.-butylbrenzkatechin |
| 4-Hydroxy-1-oxyl-2,2,6,6-tetramethylpiperidin | P(Osec-Butyl)₃ | Tert.-butylbrenzkatechin |
| 4-Hydroxy-1-oxyl-2,2,6,6-tetramethylpiperidin | P(Otert.-Butyl)₃ | Tert.-butylbrenzkatechin |
| 4-Hydroxy-1-oxyl-2,2,6,6-tetramethylpiperidin | P(OPhenyl)₃ | Tert.-butylbrenzkatechin |
| 4-Hydroxy-1-oxyl-2,2,6,6-tetramethylpiperidin | P(OH)(OMethyl)₂ | Tert.-butylbrenzkatechin |
| 4-Hydroxy-1-oxyl-2,2,6,6-tetramethylpiperidin | P(OH)(OEthyl)₂ | Tert.-butylbrenzkatechin |
| 4-Hydroxy-1-oxyl-2,2,6,6-tetramethylpiperidin | P(OH)(Oiso-Propyl)₂ | Tert.-butylbrenzkatechin |
| 4-Hydroxy-1-oxyl-2,2,6,6-tetramethylpiperidin | P(OH)(OPropyl)₂ | Tert.-butylbrenzkatechin |
| 4-Hydroxy-1-oxyl-2,2,6,6-tetramethylpiperidin | P(OH)(On-Butyl)₂ | Tert.-butylbrenzkatechin |
| 4-Hydroxy-1-oxyl-2,2,6,6-tetramethylpiperidin | P(OH)(Osec-Butyl)₂ | Tert.-butylbrenzkatechin |
| 4-Hydroxy-1-oxyl-2,2,6,6-tetramethylpiperidin | P(OH)(Otert.-Butyl)₂ | Tert.-butylbrenzkatechin |
| 4-Hydroxy-1-oxyl-2,2,6,6-tetramethylpiperidin | P(OH)(OPhenyl)₂ | Tert.-butylbrenzkatechin |
| 4-Hydroxy-1-oxyl-2,2,6,6-tetramethylpiperidin | P(OH)₂(OMethyl) | Tert.-butylbrenzkatechin |
| 4-Hydroxy-1-oxyl-2,2,6,6-tetramethylpiperidin | P(OH)₂(OEthyl) | Tert.-butylbrenzkatechin |
| 4-Hydroxy-1-oxyl-2,2,6,6-tetramethylpiperidin | P(OH)₂(Oiso-Propyl) | Tert.-butylbrenzkatechin |
| 4-Hydroxy-1-oxyl-2,2,6,6-tetramethylpiperidin | P(OH)₂(OPropyl) | Tert.-butylbrenzkatechin |
| 4-Hydroxy-1-oxyl-2,2,6,6-tetramethylpiperidin | P(OH)₂(On-Butyl) | Tert.-butylbrenzkatechin |
| 4-Hydroxy-1-oxyl-2,2,6,6-tetramethylpiperidin | P(OH)₂(Osec-Butyl) | Tert.-butylbrenzkatechin |
| 4-Hydroxy-1-oxyl-2,2,6,6-tetramethylpiperidin | P(OH)₂(Otert.-Butyl) | Tert.-butylbrenzkatechin |
| 4-Hydroxy-1-oxyl-2,2,6,6-tetramethylpiperidin | P(OH)₂(OPhenyl) | Tert.-butylbrenzkatechin |
| 4-Hydroxy-1-oxyl-2,2,6,6-tetramethylpiperidin | (Methyl)P(OMethyl)₂ | Tert.-butylbrenzkatechin |
| 4-Hydroxy-1-oxyl-2,2,6,6-tetramethylpiperidin | (Ethyl)P(OEthyl)₂ | Tert.-butylbrenzkatechin |
| 4-Hydroxy-1-oxyl-2,2,6,6-tetramethylpiperidin | (iso-Propyl)P(Oiso-Propyl)₂ | Tert.-butylbrenzkatechin |
| 4-Hydroxy-1-oxyl-2,2,6,6-tetramethylpiperidin | (Propyl)P(OPropyl)₂ | Tert.-butylbrenzkatechin |
| 4-Hydroxy-1-oxyl-2,2,6,6-tetramethylpiperidin | (n-Butyl)P(On-Butyl)₂ | Tert.-butylbrenzkatechin |
| 4-Hydroxy-1-oxyl-2,2,6,6-tetramethylpiperidin | (sec-Butyl)P(Osec-Butyl)₂ | Tert.-butylbrenzkatechin |
| 4-Hydroxy-1-oxyl-2,2,6,6-tetramethylpiperidin | (tert.-Butyl)P(Otert.-Butyl)₂ | Tert.-butylbrenzkatechin |
| 4-Hydroxy-1-oxyl-2,2,6,6-tetramethylpiperidin | (Penyl)P(OPhenyl)₂ | Tert.-butylbrenzkatechin |
| 4-Hydroxy-1-oxyl-2,2,6,6-tetramethylpiperidin | RP(OH)₂ | Tert.-butylbrenzkatechin |
| 4-Hydroxy-1-oxyl-2,2,6,6-tetramethylpiperidin | RP(OH)(OR) | Tert.-butylbrenzkatechin |
| mit R = Me, Et, iso-Pr, n-Pr, n-Bu, sec-Bu, tert-Bu, Phenyl | | |

| *Komponente a)* | *Komponente c)* | *Komponente b)* |
|---|---|---|
| 4-Hydroxy-1-oxyl-2,2,6,6-tetramethylpiperidin | P(OH)₃ | 2,6-Tert.-butyl-4-methylphenol |
| 4-Hydroxy-1-oxyl-2,2,6,6-tetramethylpiperidin | P(OMethyl)₃ | 2,6-Tert.-butyl-4-methylphenol |
| 4-Hydroxy-1-oxyl-2,2,6,6-tetramethylpiperidin | P(OEthyl)₃ | 2,6-Tert.-butyl-4-methylphenol |
| 4-Hydroxy-1-oxyl-2,2,6,6-tetramethylpiperidin | P(Oiso-Propyl)₃ | 2,6-Tert.-butyl-4-methylphenol |
| 4-Hydroxy-1-oxyl-2,2,6,6-tetramethylpiperidin | P(OPropyl)₃ | 2,6-Tert.-butyl-4-methylphenol |
| 4-Hydroxy-1-oxyl-2,2,6,6-tetramethylpiperidin | P(On-Butyl)₃ | 2,6-Tert.-butyl-4-methylphenol |
| 4-Hydroxy-1-oxyl-2,2,6,6-tetramethylpiperidin | P(Osec-Butyl)₃ | 2,6-Tert.-butyl-4-methylphenol |
| 4-Hydroxy-1-oxyl-2,2,6,6-tetramethylpiperidin | P(Otert.-Butyl)₃ | 2,6-Tert.-butyl-4-methylphenol |
| 4-Hydroxy-1-oxyl-2,2,6,6-tetramethylpiperidin | P(OPhenyl)₃ | 2,6-Tert.-butyl-4-methylphenol |
| 4-Hydroxy-1-oxyl-2,2,6,6-tetramethylpiperidin | P(OH)(OMethyl)₂ | 2,6-Tert.-butyl-4-methylphenol |
| 4-Hydroxy-1-oxyl-2,2,6,6-tetramethylpiperidin | P(OHy(OEthyl)₂ | 2,6-Tert.-butyl-4-methylphenol |
| 4-Hydroxy-1-oxyl-2,2,6,6-tetramethylpiperidin | P(OH)(Oiso-Propyl)₂ | 2,6-Tert.-butyl-4-methylphenol |
| 4-Hydroxy-1-oxyl-2,2,6,6-tetramethylpiperidin | P(OH)(OPropyl)₂ | 2,6-Tert.-butyl-4-methylphenol |
| 4-Hydroxy-1-oxyl-2,2,6,6-tetramethylpiperidin | P(OH)(On-Butyl)₂ | 2,6-Tert.-butyl-4-methylphenol |
| 4-Hydroxy-1-oxyl-2,2,6,6-tetramethylpiperidin | P(OH)(Osec-Butyl)₂ | 2,6-Tert.-butyl-4-methylphenol |
| 4-Hydroxy-1-oxyl-2,2,6,6-tetramethylpiperidin | P(OH)(Otert.-Butyl)₂ | 2,6-Tert.-butyl-4-methylphenol |
| 4-Hydroxy-1-oxyl-2,2,6,6-tetramethylpiperidin | P(OH)(OPhenyl)₂ | 2,6-Tert.-butyl-4-methylphenol |
| 4-Hydroxy-1-oxyl-2,2,6,6-tetramethylpiperidin | P(OH)₂(OMethyl) | 2,6-Tert.-butyl-4-methylphenol |
| 4-Hydroxy-1-oxyl-2,2,6,6-tetramethylpiperidin | P(OH)₂(OEthyl) | 2,6-Tert.-butyl-4-methylphenol |
| 4-Hydroxy-1-oxyl-2,2,6,6-tetramethylpiperidin | P(OH)₂(Oiso-Propyl) | 2,6-Tert.-butyl-4-methylphenol |
| 4-Hydroxy-1-oxyl-2,2,6,6-tetramethylpiperidin | P(OH)₂(OPropyl) | 2,6-Tert.-butyl-4-methylphenol |
| 4-Hydroxy-1-oxyl-2,2,6,6-tetramethylpiperidin | P(OH)₂(On-Butyl) | 2,6-Tert.-butyl-4-methylphenol |
| 4-Hydroxy-1-oxyl-2,2,6,6-tetramethylpiperidin | P(OH)₂(Osec-Butyl) | 2,6-Tert.-butyl-4-methylphenol |
| 4-Hydroxy-1-oxyl-2,2,6,6-tetramethylpiperidin | P(OH)₂(Otert.-Butyl) | 2,6-Tert.-butyl-4-methylphenol |
| 4-Hydroxy-1-oxyl-2,2,6,6-tetramethylpiperidin | P(OH)₂(OPhenyl) | 2,6-Tert.-butyl-4-methylphenol |
| 4-Hydroxy-1-oxyl-2,2,6,6-tetramethylpiperidin | (Methyl)P(OMethyl)₂ | 2,6-Tert.-butyl-4-methylphenol |
| 4-Hydroxy-1-oxyl-2,2,6,6-tetramethylpiperidin | (Ethyl)P(OEthyl)₂ | 2,6-Tert.-butyl-4-methylphenol |
| 4-Hydroxy-1-oxyl-2,2,6,6-tetramethylpiperidin | (iso-Propyl)P(OisO-Propyl)₂ | 2,6-Tert.-butyl-4-methylphenol |
| 4-Hydroxy-1-oxyl-2,2,6,6-tetramethylpiperidin | (Propyl)P(OPropyl)₂ | 2,6-Tert.-butyl-4-methylphenol |
| 4-Hydroxy-1-oxyl-2,2,6,6-tetramethylpiperidin | (n-Butyl)P(On-Butyl)₂ | 2,6-Tert.-butyl-4-methylphenol |
| 4-Hydroxy-1-oxyl-2,2,6,6-tetramethylpiperidin | (sec-Butyl)P(Osec-Butyl)₂ | 2,6-Tert.-butyl-4-methylphenol |
| 4-Hydroxy-1-oxyl-2,2,6,6-tetramethylpiperidin | (tert.-Butyl)P(Otert.-Butyl)₂ | 2,6-Tert.-butyl-4-methylphenol |
| 4-Hydroxy-1-oxyl-2,2,6,6-tetramethylpiperidin | (Penyl)P(OPhenyl)₂ | 2,6-Tert.-butyl-4-methylphenol |
| 4-Hydroxy-1-oxyl-2,2,6,6-tetramethylpiperidin | RP(OH)₂ | 2,6-Tert.-butyl-4-methylphenol |
| 4-Hydroxy-1-oxyl-2,2,6,6-tetramethylpiperidin | RP(OH)(OR) | 2,6-Tert.-butyl-4-methylphenol |
| mit R = Me, Et, iso-Pr, n-Pr, n-Bu, sec-Bu, tert-Bu, Phenyl | | |

Der besondere Vorteil der erfindungsgemäßen Inhibitorkomposition - der "Dreierkombination" - ist, daß insgesamt nur wenig Inhibitor benötigt wird.

Bei Rektifikations- und Destillationsverfahren wird vergleichsweise weniger Polymer abgeschieden, falls das erfindungsgemäße Inhibitorsystem eingesetzt wird, so daß die entsprechenden Anlagen weniger oft wegen Reinigungsarbeiten abgestellt werden müssen. Außerdem sorgt das synergistisch wirkende Inhibitorsystem dafür, daß weniger Monomer durch Polymerisation verbraucht wird und somit "verloren geht".

Selbstverständlich kann die Inhibitorkomposition neben den beschriebenen Inhibitoren - Phosphorverbindungen, Nitroxyl-Radikal(derivate), Phenol(derivate) - noch weitere eine radikalische Polymerisation inhibierende Bestandteile enthalten. Beispiele für solche sonstigen radikalischen Polymerisationsinhibitoren sind organische Nitrosoverbindungen wie N-Nitrosoarylamine. Diese Inhibitorkompositionen haben auch Wirksamkeit bei Anwesenheit von molekularem Sauerstoff.

Die Inhibitorkompositionen werden erfindungsgemäß zur Stabilisierung von Reinsubstanzen, die mindestens eine vinylisch ungesättigte Gruppe aufweisen oder von Gemischen, die wenigstens eine Substanz enthalten, welche mindestens eine vinylisch ungesättigte Gruppe aufweist, verwendet. Die Inhibitorkomposition wirkt auch in Anwesenheit von Sauerstoff.

Erfindungsgemäß wird auch ein Gemisch bereitgestellt, enthaltend wenigstens eine Verbindung, die mindestens eine vinylisch ungesättigte Gruppe aufweist und eine erfindungsgemäße Inhibitorkomposition.

Das Gemisch enthält in der Regel ein oder mehrere chemische Verbindungen, welche mindestens ein die Oxidationszahl +3 aufweisendes Phosphoratom enthalten, in einer Gesamtkonzentration von 1 bis 5000 ppm, bevorzugt von 5 bis 1000 ppm, sowie ein oder mehrere Nitroxyl-Radikal(derivate) in einer Gesamtkonzentration von 1 bis 3000 ppm, bevorzugt von 5 bis 300 ppm und ein oder mehrere Phenolderivate in einer Gesamtkonzentration von 1 bis 3000 ppm, bevorzugt von 5 bis 1500 ppm.

Unter den Verbindungen, die mindestens eine vinylisch ungesättigte Gruppe aufweisen, sind insbesondere solche zu verstehen, die sich radikalisch homo- und/oder copolymerisieren lassen. Das sind zum Beispiel Olefine, wie Isobuten, Ethylen oder Propylen, vinylaromatische Monomere wie Styrol, α-Methylstyrol, o-Chlorstyrol oder Vinyltoluole, C₄-C₈-konjugierte Diene wie Butadien oder Isopren, Ester aus Vinylalkohol und 1 bis 18 C-Atome aufweisenden Monocarbonsäuren wie Vinylacetat, Vinylpropionat, Vinyl-n-butyrat, Vinyllaurat oder Vinylstearat. Insbesondere sind auch 3 bis 6 C-Atome aufweisende α, β-monoethylenisch ungesättigte Mono- und Dicarbonsäuren, wie insbesondere Acrylsäure, Methacrylsäure, Maleinsäure, Fumarsäure und Itaconsäure, die Ester aus den vorgenannten Carbonsäuren und 1 bis 12, bevorzugt 1 bis 4 C-Atome aufweisenden Alkanolen, wie insbesondere Acrylsäure- und Methacrylsäure-methyl- -ethyl-, -n-butyl-, -iso-butyl-, -tert.-butyl- und -2-ethylhexyl-ester; Maleinsäuredimethylester oder Maleinsäuredi-n-butylester, geeignet.

Es eignen sich jedoch auch Vorläuferaldehyde, Nitrile und Amide der vorgenannten 3 bis 6 C-Atome aufweisenden α ,β-monoethylenisch ungesättigten Mono- und Dicarbonsäuren, zum Beispiel Acrolein, Methacrolein, Acrylnitril, Methacrylnitril, Acrylamid und Methacrylamid. Monomere wie Vinylsulfonsäure, Vinylphosphonsäure, N-Vinylimidazol und N-Vinylpyrrolidon kommen auch in Frage.

Die erfindungsgemäße Inhibitorkomposition ist als Zusatz sowohl zur Lagerstabilisierung als auch für die Prozeßstabilisierung (Herstellung, Reinigung und chemische Umsetzung) von wenigstens eine vinylisch ungesättigte Gruppe aufweisenden Verbindungen geeignet. Letzteres gilt insbesondere auch für destillative Prozesse, die in der Regel bei Temperaturen von 50 bis 300 °C, bevorzugt bei 50 bis 200 °C, besonders bevorzugt bei 50 bis 150 °C, ablaufen.

Insbesondere eignet sich die Stabilisierung mit der erfindungsgemäßen Inhibitorkomposition bei der destillativen (rektifikativen) Behandlung von (Meth)acrylsäureestern (insbesondere der vorgenannten beispielhaften Vertreter), bei deren destillativer oder rektifikativer Abtrennung aus Produktgemischen, wie sie als Ergebnis einer säurekatalysierten Veresterung von (Meth)acrylsäure mit Alkoholen, insbesondere Alkanolen (insbesondere C₁- bis C₁₂- bzw. C₁- bis C₈-Alkanolen) vor und/oder nach Abtrennung des Säurekatalysators vorliegen.

Diese eignet sich aber auch zur Stabilisierung von vorgenannten (Meth)-acrylsäureester enthaltenden Gemischen, die weder Veresterungskatalysator noch Acryl- oder Methacrylsäure selbst enthalten. Solche (Meth)acrylsäureester enthaltende Gemische bilden beispielsweise vorgenannte Veresterungsprodukt-gemische nach zum Beispiel extraktiver und/oder rektifikativer Abtrennung des Säurekatalysators sowie nach entsprechender Abtrennung der überschüssigen (Meth)acrylsäure.

Die Stabilisierung eines einer Destillation oder Rektifikation unterworfenen (Meth)acrylsäureester enthaltenden Gemisches kann so erfolgen, daß man die Inhibitoren dem Gemisch bereits vor der Destillation zusetzt.

Zusätzlich kann zur Stabilisierung eine Inhibitorzugabe auf den Kopf der Kolonne erfolgen. Selbstverständlich kann auch die gesamte Stabilisierung ausschließlich durch eine Inhibitorzugabe auf den Kolonnenkopf erfolgen.

Die verschiedenen Komponenten der erfindungsgemäßen Inhibitorkomposition können zeitlich nacheinander, simultan oder auch bereits vorgemischt zugesetzt werden. Das vorgenannte gilt auch für die anderen Inhibitoren, falls das Inhibitorkomposition solche umfaßt.

Auch kann die Zugabe der Komponenten der Inhibitorkomposition an unterschiedlichen Zugabeorten vorgenommen werden. So können zum Beispiel Komponenten des Inhibitorsystems am Kopf der Rektifikationskolonne und andere Komponenten des Inhibitorsystems in den Sumpf und/oder den Zulauf der Rektifikationskolonne gegeben werden. Dies gilt sowohl für solche Retifikationen im Rahmen derer der (Meth)acrylsäureester über Kopf-, über Sumpf- und/oder über Seitenabzug abgetrennt wird. Auch kann es zweckmäßig sein, das erfindungsgemäße Verfahren im Fall einer kontinuierlichen destillativen (retifikativen) Abtrennung von (Meth)acrylsäureestern so durchzuführen, daß wenigstens eine zuzuführende Inhibitorkomponente nicht kontinuierlich, sondern lediglich von Zeit zu Zeit, d.h. periodisch wiederkehrend, zugegeben wird (z.B. am Kolonnenkopf, im Sumpf und/oder im Zulauf).

Alles über die Stabilisierung bei der destillativen (rektifikativen) Abtrennung von (Meth)acrylsäureestern aus säurekatalysierten Veresterungsgemischen beschriebene, gilt in gleicher Weise auch bezüglich einer destillativen (rektifikativen) Abtrennung von (Meth)acrylsäure bzw. (Meth)acrolein aus diese enthaltenden Gemischen.

Unter anderem ist (Meth)acrylsäure durch katalytische Gasphasenoxidation von Alkanen, Alkanolen, Alkenen oder Alkenalen erhältlich, die 3 bzw. 4 C-Atome enthalten. Besonders vorteilhaft ist (Meth)acrylsäure z.B. durch katalytische Gasphasenoxidation von Propan, Propen, tert.-Butanol, iso-Buten, iso-Butan, iso-Butyraldehyd oder Methacrolein erhältlich. Als Ausgangsverbindungen sind aber auch solche denkbar, aus welchen sich die eigentliche C₃-/C₄-Ausgangsver-bindung während der Gasphasenoxidation erst intermediär bildet. Beispielhaft genannt sei der Methylether des tert.-Butanols.

Dabei werden diese Ausgangsgase, in der Regel mit Inertgasen wie Stickstoff, CO, CO₂, gesättigten Kohlenwasserstoffen und/oder Wasserdampf verdünnt, im Gemisch mit Sauerstoff bei erhöhten Temperaturen (üblicherweise 200 bis 400°C) sowie gegebenenfalls erhöhtem Druck über übergangsmetallische (z.B. Mo, V, W und/oder Fe enthaltende) Mischoxidkatalysatoren geleitet und oxidativ in die (Meth)acrylsäure umgewandelt.

Aufgrund zahlreicher im Verlauf der katalytischen Gasphasenoxidation erfolgender Parallel- und Folgereaktionen sowie aufgrund der mitzuver-wendenden inerten Verdünnungsphase wird bei der katalytischen Gasphasenoxidation jedoch keine reine (Meth)acrylsäure, sondern ein Reaktionsgemisch erhalten, das im wesentlichen (Meth)acrylsäure, die inerten Verdünnungsgase und Nebenprodukte enthält, aus welchem die (Meth)acrylsäure abgetrennt werden muß. Neben von (Meth)acrylsäure vergleichsweise einfach zu entfernenden und bei Folgeverwendungen der (Meth)acrylsäure weniger störenden Nebenprodukten wie Essigsäure, enthält das Reaktionsgasgemisch häufig auch mit (Meth)acrylsäure eng verwandte und daher von (Meth)acrylsäure schwer abtrennbare niedere Aldehyde wie Formaldehyd, Acetaldehyd, Acrolein, Methacrolein, Propionaldehyd, n-Butyraldehyd, Benzaldehyd, Furfural und Crotonaldehyd sowie zusätzlich gegebenenfalls Maleinsäureanhydrid (bezogen auf die im Reaktionsgasgemisch enthaltende Menge an (Meth)acrylsäure beträgt die Gesamtmenge dieser, bei Folgeverwendungen häufig erheblich störenden, Nebenkomponenten in der Regel ≤ 2 Gew.-%, meist ≥ 0,05 Gew.-%).

Es ist außerdem praktikabel, Acrylsäure aus dem Reaktionsgasgemisch der katalytischen Gasphasenoxidation von Propylen und/oder Acrolein durch Gegenstromadsorption mit einer hochsiedenden inerten hydrophoben organischen Flüssigkeit abzutrennen. Die Inhibitorkomposition kann dabei direkt in den Gasstrom bzw. in die Vorrichtung zur Gegenstromadsorption eingespritzt werden. Als organische Flüssigkeit für die Gegenstromadsorption kommen dabei u.a. höhere Alkohole oder Ester dieser (insbesondere solche mit (Meth)acrylsäure), in Frage. Das Verfahren wird im wesentlichen so durchgeführt, daß man das Reaktionsgasgemisch in einer konventionellen Adsorptionskolonne zu der absteigenden Adsorptionsflüssigkeit im Gegenstrom führt, danach in einer Desorptionskolonne aus dem im wesentlichen aus Acrylsäure, dem Adsorptionsmittel und Nebenkomponenten zusammmengesetzten Flüssigkeitsablauf der Adsorptionskolonne durch Strippen (Abstreifen) mit Inertgas die einfach abtrennbaren leichtflüchtigen Nebenkomponenten weitgehend entfernt und anschließend den (Meth)acrylsäure und das Adsorbens als Hauptbestandteile enthaltenden Flüssigkeitsablauf der Desorptionskolonne zur Abtrennung von Roh-Acrylsäure rektifikativ behandelt.

Das Problem einer rektifikativen Abtrennung von (Meth)acrylsäure erwächst auch dann, wenn man die (Meth)acrylsäure aus den Reaktionsgasen der katalytischen Gasphasenoxidation zunächst in Wasser aufnimmt und anschließend unter Zusatz eines organischen azeotropen Schleppers aus den wäßrigen (Meth)acrylsäure enthaltenden Gemischen das Wasser rektifikativ abtrennt.

Das Problem besteht aber auch bei der rektifikativen Herstellung von Reinacrylsäure (Reinheit > 99,7 Gew.-%) aus Roh-Acrylsäure (Reinheit > 99 Gew.-%).

Bei allen diesen vorgenannten Rektifikationsproblemen ist die erfindungsgemäße Inhibitorkomposition anwendbar. Selbstverständlich kann die erfindungsgemäße Inhibitorkomposition auch bei der Extraktion der (Meth)acrylsäure aus dem Reaktionsgemisch der Gasphasenoxidation eingesetzt werden. Die Inhibitorkomposition kann dabei direkt in den Gasstrom eingespritzt werden. Die Stabilisierung empfiehlt sich auch für den Fall einer kristallisativen Abtrennung von (Meth)acrylsäure oder deren Ester enthaltenden Gemischen.

(Meth)acrolein ist in entsprechender Weise wie (Meth)acrylsäure z.B. durch katalytische Gasphasenoxidation erhältlich. Allerdings wird die Oxidation nach der ersten Oxidationsstufe nicht weitergeführt. Vielmehr wird das im Reaktionsgasgemisch enthaltende (Meth)acrolein in der Regel zunächst mittels Wasser extraktiv aus dem Reaktionsgasgemisch abgetrennt und anschließend destillativ (rektifikativ) aus der wäßrigen Lösung gewonnen. Für alle genannten Prozeßschritte eignet sich zur Stabilisation die erfindungsgemäße Inhibitorkomposition.

Die synergistische Wirksamkeit der erfindungsgemäßen Inhibitorkomposition gilt im wesentlichen unabhängig vom pH-Wert und sowohl für niedere (z.B. Raumtemperatur) als auch für erhöhte Temperaturen, wie sie z. B. im Rahmen von thermischen physikalischen Trennverfahren als auch für bei erhöhten Temperaturen ablaufende chemische Umsetzungen üblich sind.

Insbesondere gilt das vorgenannte für eine Stabilisierung von (Meth)acrylsäure und/oder deren Ester, deren ethylenisch ungesättigte Doppelverbindung bezüglich einer radikalischen Polymerisation besonders aktiv ist.

In der Regel wird die Inhibitorkomposition so gewählt, daß die in der Inhibitorkomposition enthaltenen Komponenten in der zu stabilisierenden Substanz in ihrer Einsatzmenge in vollem Umfang löslich sind. Häufig erfolgt ihre Zugabe nicht als Reinsubstanz, sondern als Suspension, Emulsion oder Lösung. Als Lösungs- und/oder Dispergiermedium kommen insbesondere diejenigen Substanzen in Betracht, die Bestandteil des zu stabilisierenden Systems sind, d.h., z.B. bei chemischen Umsetzungen wie Veresterungen alle Edukte und Produkte.

Im folgenden soll die Erfindung anhand eines Ausführungsbeispiels näher erläutert werden.

### Beispiel:

1ml Acrylsäure wurde jeweils mit verschiedenen Stabilisatoren unterschiedlicher Menge versetzt und in Glasampullen (Innenvolumen 1,9 ml) unter Luft gasdicht eingeschlossen. Die Ampullen wurden vollständig in ein 120°C heißes Ölbad eingetaucht und unter Lichtausschluß gelagert.

Anschließend wurde die Zeit bestimmt, bis die Acrylsäure auspolymerisiert war. Dabei wurde visuell der Zeitpunkt ermittelt, bei dem man die Verfestigung der Polymerisationsmischung erkennen konnte. Die nachstehende Tabelle zeigt die zugesetzten Mengen der Komponenten des Inhibitors und entsprechend die Zeit bis zum Festwerden der entsprechenden Polymerisationsmischung.

| **Versuchs-Nr.** | **HO-TEMPO (ppm)** | **H**_{**3**}**PO**_{**3**} **ppm** | **MEHQ ppm** | **Zeit (min)** |
|---|---|---|---|---|
| 1 | 10 | | | 77 |
| 2 | | 25 | | 16,5 |
| 3 | | | 50 | 55 |
| 4 | | 25 | 50 | 71,5 |
| 5 | 10 | 25 | | 110 |
| 6 | 10 | | 50 | 214 |
| 7 | 10 | 25 | 50 | 319 |
| Angaben jeweils in Gew.-ppm bezogen auf die zugesetzte Acrylsäure HO-Tempo: 4-Hydroxy-1-oxyl-2,2,6,6-tetramethylpiperidin MEHQ: Hydrochinonmonomethylether | | | | |

Die Versuchsergebnisse zeigen, daß bei Verwendung aller drei Inhibitortypen - also Verbindungen, die ein Phosphoratom in der Oxidationszahl +3 aufweisen, sowie Nitroxyl-Radikal(derivate) und Phenol(derivate) - eine besonders lange Zeit bis zum Festwerden der entsprechenden Probe zu verzeichnen ist. Das zeigt, daß die synergistische Wirkung (die gegenseitige Steigerung der inhibierenden Wirkung der einzelnen Komponenten) dieser "Dreierkomposition" besonders intensiv ist.

## Patentansprüche

1. Inhibitorkomposition enthaltend als Komponenten a) wenigstens ein Nitroxyl-Radikal(derivat), b) wenigstens ein Phenol(derivat) und c) wenigstens eine weitere chemische Verbindung, welche mindestens ein Phosphoratom enthält, das die Oxidationszahl +3 aufweist.

2. Inhibitorkomposition nach Anspruch 1, **dadurch gekennzeichnet, daß** als Nitroxyl-Radikal(derivat) 4-Hydroxy-1-oxyl-2,2,6,6-tetramethylpiperidin eingesetzt wird.

3. Inhibitorkomposition nach Anspruch 1 oder 2, **dadurch gekennzeichnet, daß** als Verbindung, die mindestens ein Phosphoratom enthält, Orthophosphorige Säure oder ein Ester der Orthophosphorigen Säure eingesetzt wird.

4. Inhibitorkomposition nach einem der Ansprüche 1 bis 3, **dadurch gekennzeichnet, daß** als Phenol(derivat) ein Phenol oder Kresol, bei dem ein oder mehrere Wasserstoffatome durch tert.-Butyl-Gruppen substituiert sind, insbesondere 2,6-Ditert.-butylkresol oder Hydrochinone, insbesondere Hydrochinonmonomethylether, eingesetzt wird.

5. Gemisch enthaltend wenigstens eine Verbindung, die mindestens eine vinylisch ungesättigte Gruppe aufweist und eine Inhibitorkomposition gemäß einem der Ansprüche 1 bis 4.

6. Gemisch nach Anspruch 5 enthaltend als Inhibitorkomposition ein oder mehrere chemische Verbindungen, welche mindestens ein die Oxidationszahl +3 aufweisendes Phosphoratom enthalten, in einer Gesamtkonzentration von 1 bis 5000 ppm, bevorzugt von 5 bis 1000 ppm, sowie ein oder mehrere Nitroxyl-Radikal(derivate) in einer Gesamtkonzentration von 1 bis 3000 ppm, bevorzugt von 5 bis 300 ppm und ein oder mehrere Phenolderivate in einer Gesamtkonzentration von 1 bis 3000 ppm, bevorzugt von 5 bis 1500 ppm.

7. Verwendung von Inhibitorkompositionen gemäß einem der Ansprüche 1 bis 4 zur Stabilisierung von Reinsubstanzen, die mindestens eine vinylisch ungesättigte Gruppe aufweisen oder von Gemischen, die wenigstens eine Substanz enthalten, welche mindestens eine vinylisch ungesättigte Gruppe aufweist.

## Claims

1. An inhibitor composition containing, as components, a) at least one nitroxyl radical (derivative), b) at least one phenol (derivative) and c) at least one further chemical compound which contains at least one phosphorus atom which has the oxidation state +3.

2. An inhibitor composition as claimed in claim 1, wherein the nitroxyl radical (derivative) used is 4-hydroxy-1-oxyl-2,2,6,6-tetramethylpiperidine.

3. An inhibitor composition as claimed in claim 1 or 2, wherein orthophosphorous acid or an ester of orthophosphorous acid is used as the compound which contains at least one phosphorus atom.

4. An inhibitor composition as claimed in any of claims 1 to 3, wherein the phenol (derivative) used is a phenol or cresol in which one or more hydrogen atoms have been substituted by tert-butyl groups, in particular 2,6-di-tert-butylcresol, or hydroquinones, in particular hydroquinone monomethyl ether.

5. A mixture containing at least one compound which has at least one vinylically unsaturated group and an inhibitor composition as claimed in any of claims 1 to 4.

6. A mixture as claimed in claim 5, containing, as an inhibitor composition, one or more chemical compounds which contain at least one phosphorus atom having the oxidation state +3, in a total concentration of from 1 to 5000 ppm, preferably from 5 to 1000 ppm, and one or more nitroxyl radical (derivatives) in a total concentration of from 1 to 3000 ppm, preferably from 5 to 300 ppm, and one or more phenol derivatives in a total concentration of from 1 to 3000 ppm, preferably from 5 to 1500 ppm.

7. The use of an inhibitor composition as claimed in any of claims 1 to 4 for stabilizing pure substances which have at least one vinylically unsaturated group or of mixtures which contain at least one substance which has at least one vinylically unsaturated group.

## Revendications

1. Composition inhibitrice contenant, en tant que composants a) au moins un radical nitroxyle (ou un dérivé de celui-ci), b) au moins un phénol (ou un dérivé de celui-ci) et c) au moins un autre composé chimique, qui contient au moins un atome de phosphore, qui présente l'état d'oxydation +3.

2. Composition inhibitrice selon la revendication 1, **caractérisée en ce qu'**on utilise en tant que radical nitroxyle (ou dérivé de celui-ci) de la 4-hydroxy-1-oxyl-2,2,6,6-tétraméthylpipéridine.

3. Composition inhibitrice selon la revendication 1 ou 2, **caractérisée en ce qu'**on utilise en tant que composé contenant au moins un atome de phosphore un acide orthophosphoreux ou un ester de l'acide orthophosphoreux.

4. Composition inhibitrice selon l'une quelconque des revendications 1 à 3, **caractérisée en ce qu'**on utilise en tant que phénol (ou dérivé de celui-ci) un phénol ou un crésol, dans lequel un ou plusieurs atomes d'hydrogène sont substitués par des groupements tert-butyle, en particulier le 2,6-di-tert-butylcrésol ou des hydroquinones, en particulier l'hydroquinonemonométhyléther.

5. Mélange contenant au moins un composé, qui présente au moins un groupe vinyliquement insaturé et une composition inhibitrice selon l'une quelconque des revendications 1 à 4.

6. Mélange selon la revendication 5, contenant en tant que composition inhibitrice un ou plusieurs composés chimiques, qui contiennent au moins un atome de phosphore présentant l'état d'oxydation +3, en une concentration totale de 1 à 5000 ppm, de préférence de 5 à 1000 ppm, ainsi qu'un ou plusieurs radicaux nitroxyle (ou dérivés de ceux-ci) en une concentration totale de 1 à 3000 ppm, de préférence de 5 à 300 ppm et un ou plusieurs dérivés du phénol en une concentration totale de 1 à 3000 ppm, de préférence de 5 à 1500 ppm.

7. Utilisation de compositions inhibitrices selon l'une quelconque des revendications 1 à 4 pour la stabilisation de substances pures qui présentent au moins un groupe vinyliquement insaturé ou de mélanges qui contiennent au moins une substance qui présente au moins un groupe vinyliquement insaturé.
